(19) Europäisches Patentamt
European Patent Office
Office européen des brevets

(11) **EP 4 248 835 A1**

(12) **EUROPEAN PATENT APPLICATION**
published in accordance with Art. 153(4) EPC

(43) Date of publication:
**27.09.2023 Bulletin 2023/39**

(51) International Patent Classification (IPC):
**A61B 1/06** (2006.01)

(21) Application number: **21894049.2**

(22) Date of filing: **19.11.2021**

(86) International application number:
**PCT/CN2021/131950**

(87) International publication number:
**WO 2022/105902 (27.05.2022 Gazette 2022/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.11.2020 CN 202011309665**

(71) Applicant: **Shanghai Microport Medbot (Group) Co., Ltd.**
**Shanghai 201203 (CN)**

(72) Inventors:
• **LIANG, Xiangnan**
  **Shanghai 201203 (CN)**
• **MAO, Haoyang**
  **Shanghai 201203 (CN)**
• **CAO, Lun**
  **Shanghai 201203 (CN)**
• **HE, Yuyuan**
  **Shanghai 201203 (CN)**
• **HE, Chao**
  **Shanghai 201203 (CN)**

(74) Representative: **Patentship Patentanwaltsgesellschaft mbH Elsenheimerstraße 65 80687 München (DE)**

(54) **FLUORESCENCE ENDOSCOPE SYSTEM, CONTROL METHOD AND STORAGE MEDIUM**

(57) A fluorescence endoscopy system has operating modes including a first mode and a second mode. The fluorescence endoscopy system includes an endoscope (100), an illumination module (200), an endoscope drive module (300) and a scene fusion module (500). The endoscope (100) includes a visible-light image sensor (111) and a near-infrared image sensor (121). In the second mode, the illumination module (200) is configured to provide visible light and near-infrared light. The visible-light image sensor (111) captures visible-light scene images and outputs them in the form of a first video stream, and the near-infrared image sensor (121) captures near-infrared scene images and outputs them in the form of a second video stream. The scene fusion module (500) is configured to, when current brightness value(s) of the first and/or second video stream(s) lie(s) with respective target brightness range(s), a current image frame of the first video stream is fused with a current image frame of the second video stream. Also disclosed is a control method for a fluorescence endoscopy system and a storage medium. The method can achieve an effectively increased signal-to-noise ratio, which allows more details to be discerned and obtained from the captured images.

Fig. 1

**Description**

**TECHNICAL FIELD**

The present invention relates to the field of medical devices and, in particular, to a fluorescence endoscopy system, a control method and a storage medium.

**BACKGROUND**

As medical technology advances, endoscopes, detection instruments that integrate traditional optics, ergonomics, precision machinery, modern electronics, mathematics, software and other technology, are finding increasingly extensive application. An endoscope can be inserted into the body of a subject to be examined (e.g., into his/her esophagus) to capture images of a site of interest, from which whether there is a lesion at the site can be determined. Endoscopes are very useful for physicians because they can be used to identify lesions that cannot be detected by X-rays. For example, with the aid of an endoscope, a physician can observe a stomach ulcer or tumor and derive the best possible treatment plan from the observation.

Specifically, an endoscopy system typically includes a component that can be inserted into the body of a living being through the mouth or another natural orifice, a surgically made incision or the like. After being inserted, this component can capture image information of the interior of the body of the living being and transmit the information to a monitor disposed out of the body for display of the captured images thereon.

Generally, an endoscopy system is capable of ordinary light (visible light) imaging. As the name suggests, ordinary light imaging of an object within the body of a living being is carried out under illumination with ordinary light or white light. For example, an endoscopy system may successively apply light beams of the three colors R, G and B to tissue of interest in the body of a living being and produce an ordinary light image from images formed by reflections of those light beams. Although such ordinary light images can help physicians make diagnostic determinations, they suffer from some limitations. For example, some lesions such as squamous cell carcinoma are difficult to visually identify in ordinary light images. As another example, in endometrial cancer surgery, it is difficult to identify a sentinel lymph node in an ordinary light image.

For this reason, endoscopy systems utilizing special light (e.g., fluorescence) for imaging have been developed. These systems can provide observers with information indiscernible with ordinary light imaging, providing more abundant diagnostic and therapeutic aids. For example, an image of tissue of interest in relation to endometrial cancer captured using a special light imaging technique can show a strong contrast between a sentinel lymph node and the surrounding normal tissue (e.g., one of them may be displayed almost as a white area and the other almost as a black area in a processed form of the image). This enables easy distinction of the two types of tissue. However, it is just the strong contrast between the cancerous and normal tissue (e.g., one of them may be displayed almost as a white area and the other almost as a black area in a processed form of the image) that obscures structural and morphological details of the tissue of interest in the special light image. In order to avoid this, a fluorescence endoscopy system typically captures both ordinary and special light images, which are then displayed side-by-side or overlaid upon one another.

When bleeding occurs in a cavity during a surgical procedure, the brightness therein will decrease due to the light-absorbing properties of hemoglobin, obscuring details in the captured images. Conventionally, this has been coped with by increasing the endoscope's exposure time and gain. However, this approach would lead to smear or aggravated image noise.

**SUMMARY OF THE INVENTION**

It is an object of the present invention to provide a fluorescence endoscopy system, a control method and a storage medium capable of imaging particular tissue with fluorescence and obtaining information that cannot be obtained with visible light. Moreover, they circumvent the problems of smear and aggravated image noise that may arise from the use of a conventional method of fusion of successive frames of captured images for addressing obscured details in the images in the event of bleeding occurring in a surgical procedure performed in an environment illuminated with visible light.

To this end, the present invention provides a fluorescence endoscopy system comprising an endoscope, an illumination module, an endoscope drive module and a scene fusion module.

Operating modes of the fluorescence endoscopy system include a first mode and a second mode.

The endoscope comprises a visible-light image sensor and a near-infrared image sensor.

The illumination module is configured to provide visible light for illuminating the tissue of interest and excitation light for exciting the tissue of interest to produce fluorescence in the first mode, where the visible-light image sensor is configured to capture visible-light scene images of the tissue of interest and output them in the form of a first video stream, and the near-infrared image sensor is configured to capture fluorescence scene images of the tissue of interest and output them in the form of a second video stream.

The illumination module is configured to provide visible light and near-infrared light both for illuminating the tissue of interest in the second mode, where the visible-light image sensor is configured to capture visible-light scene images of the tissue of interest and output them in the form of a first video stream, and the near-infrared image sensor is configured to capture near-infrared scene images of the tissue of interest and output them in the form of a second video stream.

The endoscope drive module comprises a first drive unit and a second drive unit. The first drive unit is configured to drive the visible-light image sensor to capture the visible-light scene images according to a first exposure time and a first gain in the second mode, and the second drive unit is configured to drive the near-infrared image sensor to capture the near-infrared scene images according to a second exposure time and a second gain in the second mode.

The scene fusion module is configured to, when a current brightness value of the first video stream lies within a predefined first target brightness range and/or a current brightness value of the second video stream lies within a predefined second target brightness range, obtain a brightness-fused image by fusing a current image frame in the first video stream with a current image frame in the second video stream based on brightness information of the current image frame in the first video stream and brightness information of the current image frame in the second video stream and obtain a scene-fused image by fusing the brightness-fused image with the current image frame in the first video stream based on chroma information of the current image frame in the first video stream.

Optionally, the fluorescence endoscopy system may further comprise an endoscope control module, the endoscope control module comprising a first control unit and/or a second control unit, the first control unit configured to cause the current brightness value of the first video stream to lie within the predefined first target brightness range in the second mode, the second control unit configured to cause the current brightness value of the second video stream to lie within the predefined second target brightness range in the second mode.

Optionally, the first control unit may comprise:

a first brightness acquisition means for acquiring the current brightness value of the first video stream; and
a first exposure control means for determining whether the current brightness value of the first video stream lies within the first target brightness range and, if the current brightness value of the first video stream does not lie within the first target brightness range, adjusting a first exposure amount of the visible-light image sensor to cause the current brightness value of the first video stream to lie within the first target brightness range.

Additionally, the second control unit may comprise:

a second brightness acquisition means for acquiring the current brightness value of the second video stream; and
a second exposure control means for determining whether the current brightness value of the second video stream is within the second target brightness range and, if the current brightness value of the second video stream does not lie within the second target brightness range, adjusting a second exposure amount of the near-infrared image sensor to cause the current brightness value of the second video stream to lie within the second target brightness range.

Optionally, when the current brightness value of the first video stream is below a lower limit of the first target brightness range, the first exposure control means may adjust the current brightness value of the first video stream by increasing the first exposure amount of the visible-light image sensor.

Additionally, when the current brightness value of the second video stream is below a lower limit of the second target brightness range, the second exposure control means may adjust the current brightness value of the second video stream by increasing the second exposure amount of the near-infrared image sensor.

Optionally, the first exposure control means may be configured to: determine whether a maximum value of the first exposure time and a minimum value of the first gain satisfy an exposure amount requirement of the first target brightness range;

if the requirement is satisfied, with the first gain being kept at the minimum value, adjust the first exposure time to cause the current brightness value of the first video stream to lie within the first target brightness range; and
if the requirement is not satisfied, determine whether the maximum value of the first exposure time and a maximum value of the first gain satisfy the exposure amount requirement of the first target brightness range and,
if so, with the first exposure time being kept at the maximum value, adjust the first gain to cause the current brightness value of the first video stream to lie within the first target brightness range.

Additionally, the second exposure control means may be configured to: determine whether a maximum value of the second exposure time and a minimum value of the second gain satisfy an exposure amount requirement of the second target brightness range;

if the requirement is satisfied, with the second gain being kept at the minimum value, adjust the second exposure time to cause the brightness value of the second video stream to lie within the second target brightness range; and
if the requirement is not satisfied, determine whether the maximum value of the second exposure time and a maximum value of the second gain satisfy the exposure amount requirement of the second target brightness range and,

if so, with the second exposure time being kept at the maximum value, adjust the second gain to cause the brightness value of the second video stream to lie within the second target brightness range.

Optionally, when the current brightness value of the first video stream is above an upper limit of the first target brightness range, the first exposure control means may adjust the current brightness value of the first video stream by reducing the first exposure amount of the visible-light image sensor.

Additionally, when the current brightness value of the second video stream is above an upper limit of the second target brightness range, the second exposure control means may adjust the current brightness value of the second video stream by reducing the second exposure amount of the near-infrared image sensor.

Optionally, the first exposure control means may be configured to: determine whether a maximum value of the first exposure time and a minimum value of the first gain satisfy an exposure amount requirement of the first target brightness range;

if the requirement is satisfied, with the first exposure time being kept at the maximum value, adjust the first gain to cause the current brightness value of the first video stream to lie within the first target brightness range; and

if the requirement is not satisfied, determine whether a minimum value of the first exposure time and the minimum value of the first gain satisfy the exposure amount requirement of the first target brightness range and,

if so, with the first gain being kept at the minimum value, adjust the first exposure time to cause the current brightness value of the first video stream to lie within the first target brightness range.

Additionally, the second exposure control means may be configured to: determine whether a maximum value of the second exposure time and a minimum value of the second gain satisfy an exposure amount requirement of the second target brightness range;

if the requirement is satisfied, with the second exposure time being kept at the maximum value, adjust the second gain to cause the brightness value of the second video stream to lie within the second target brightness range; and

if the requirement is not satisfied, determine whether a minimum value of the second exposure time and the minimum value of the second gain satisfy the exposure amount requirement of the second target brightness range and,

if so, with the second gain being kept at the minimum value, adjust the second exposure time to cause the brightness value of the second video stream to lie within the second target brightness range.

Optionally, the illumination module may comprise a first light source module for providing the visible light and a third light source module for providing the near-infrared light, wherein the first control unit further comprises a first illumination adjustment means for controlling, when it is impossible to cause the current brightness value of the first video stream to lie within the first target brightness range by adjusting the first gain and the first exposure time, the illumination module to adjust output power of the first light source module to cause the current brightness value of the first video stream to lie within the first target brightness range, and wherein the second control unit further comprises a second illumination adjustment means for controlling, when it is impossible to cause the current brightness value of the second video stream to lie within the second target brightness range by adjusting the second gain and the second exposure time, the illumination module to adjust output power of the third light source module to cause the current brightness value of the second video stream to lie within the second target brightness range.

Optionally, in case of the first video stream being YUV or YCbCr- coded, the first brightness acquisition means may be configured to take an average or weighted value of Y values of all or some pixels of the current image frame of the first video stream as the current brightness value.

Additionally, in case of the first video stream being first video stream being RAW- or RGB-coded, the first brightness acquisition means may be configured to derive brightness values of the pixels of the current image frame of the first video stream from RGB values thereof and then take an average or weighted value of the brightness values of all or some pixels of the current image frame of the first video stream as the current brightness value.

Optionally, the endoscope may further comprise a beam-splitting prism group for separating the visible light reflected from the illuminated tissue of interest from the near-infrared light reflected from the illuminated tissue of interest in the second mode and separating the visible light reflected from the illuminated tissue of interest from the fluorescence produced by the excited tissue of interest in the first mode so that the reflected visible light can be captured by a photosensitive surface of the visible-light image sensor and that the reflected near-infrared light or the fluorescence can be captured by a photosensitive surface of the near-infrared image sensor.

Optionally, the beam-splitting prism group may comprise a first beam-splitting prism, a second beam-splitting prism, a visible-light band-pass filter and a near-infrared band-pass filter, the visible-light band-pass filter configured to allow passage of visible light therethrough and block light at other wavelengths, the near-infrared band-pass filter configured to allow passage of near-infrared light therethrough and block light at other wavelengths, the near-infrared band-pass filter disposed between the first beam-splitting prism and the second beam-splitting prism, the first beam-splitting prism provided with a semi-transmissive, semi-reflective film on a surface thereof adjacent to the second beam-splitting prism, which reflects part of light incident thereon and allows transmission of the rest of the incident light, wherein: the photosensitive surface of the visible-light image sensor is adjacent to an exit surface of the first beam-splitting prism; the visible-light band-pass filter is disposed between the exit surface of the first beam-splitting prism and the photosensitive surface of the visible-light image sensor; and the photosensitive surface of the near-infrared image sensor is adjacent to an exit surface of the second beam-splitting prism.

Optionally, the scene fusion module may comprise:

an image fusion unit for obtaining the brightness-fused image by fusing pixels of the current image frame in the first video stream with respective pixels of the current image frame in the second video stream based on the brightness information of the current image frame in the first video stream and the brightness information of the current image frame in the second video stream and obtaining the scene-fused image by fusing the brightness-fused image with the current image frame in the first video stream based on the chroma information of the current image frame in the first video stream.

Optionally, the image fusion unit may be configured to: derive weights for brightness values of the pixels of the current image frame in the first video stream and weights for brightness values of the respective pixels of the current image frame in the second video stream from the brightness values of the pixels of the current image frame in the first video stream and the brightness values of the respective pixels of the current image frame in the second video stream, respectively, according to a predefined normal weight distribution as a function of brightness; and

obtain brightness value of pixels of the brightness-fused image by weighting the brightness values of the pixels of the current image frame in the first video stream and the brightness values of the respective pixels of the current image frame in the second video stream with the derived weights for the brightness values of the pixels of the current image frame in the first video stream and weights for the brightness values of the respective pixels of the current image frame in the second video stream, respectively.

Optionally, the scene fusion module may further comprise an image mode switching unit for converting, in case of the first video stream being output in a RAW or RGB format, pixels of the current image frame in the first video stream into the YUV or YCbCr space and taking Y values of the pixels of the current image frame in the first video stream as brightness values of the pixels and U or V values or Cb or Cr values of the pixels of the current image frame in the first video stream as chroma values of the pixels of the current image frame in the first video stream.

Optionally, the endoscope may a three-dimensional (3D) endoscope, wherein:

the visible-light image sensor comprises a first visible-light image sensor and a second visible-light image sensor;
the near-infrared image sensor comprises a first near-infrared image sensor and a second near-infrared image sensor;
the first video stream comprises a first visible-light video stream and a second visible-light video stream;
the second video stream comprises a first near-infrared video stream and a second near-infrared video stream;
in the second mode, the first visible-light image sensor is configured to capture first visible-light scene images of the tissue of interest and output them in the form of a first visible-light video stream, the second visible-light image sensor is configured to capture second visible-light scene images of the tissue of interest and output them in the form of a second visible-light video stream, the first near-infrared image sensor is configured to capture first near-infrared scene images of the tissue of interest and output them in the form of a first near-infrared video stream, and the second near-infrared image sensor is configured to capture second near-infrared scene images of the tissue of interest and output them in the form of a second near-infrared video stream; and
the scene fusion module is configured to obtain: a first brightness-fused image by fusing a current image frame in the first visible-light video stream with a current image frame in the first near-infrared video stream based on brightness information of the current image frame in the first visible-light video stream and brightness information of the current image frame in the first near-infrared video stream; a first scene-fused image by fusing the first brightness-fused image with the current image frame in the first visible-light video stream based on chroma information of the current image frame in the first visible-light video stream; a second brightness-fused image by fusing a current image frame in the second visible-light video stream with a current image frame in the second near-infrared video stream based on brightness information of the current image frame in the second visible-light video stream and the brightness information of the current image frame in the second near-infrared video stream; and a second scene-fused image by fusing the second brightness-fused image with the current image frame in the second visible-light video stream based on chroma information of the current image frame in the second visible-light video stream.

Optionally, the fluorescence endoscopy system may further comprise a central controller, which, upon receiving a command for activating the first mode, controls the first control unit and the second control unit to adjust, according to the first target brightness range and the second target brightness range that are pre-configured in the endoscope control module, the current brightness values of the first video stream and the second video stream, or transmits the first target brightness range and the second target brightness range to the first control unit and the second control unit, respectively, so that the first control unit and the second control unit adjust, according to the first target brightness range and the second target brightness range that are pre-configured in the endoscope control module, the current brightness values of the first video stream and the second video stream.

Optionally, the fluorescence endoscopy system may further comprise a central controller, the central controller comprising a video overlay unit for producing a 3D image by overlaying scene-fused images output from the scene fusion module and transmitting the produced 3D image to a monitor for its display thereon.

Optionally, the first drive unit may be further configured to drive the visible-light image sensor to capture the visible-light scene images according to a third exposure time and a third gain in the first mode, and the second drive unit may be further configured to drive the near-infrared image sensor to capture the fluorescence scene images according to a fourth exposure time and a fourth gain in the first mode.

Optionally, the first control unit may be further configured to cause the current brightness value of the first video stream to lie within a predefined third target brightness range in the first mode. Alternatively or additionally, the second control unit may be further configured to cause the current brightness value of the second video stream to lie within a predefined fourth target brightness range in the first mode.

To the above end, the present invention also provides a control method of a fluorescence endoscopy system. The fluorescence endoscopy system includes a first mode and a second mode. The method comprises:

in the second mode, providing visible light and near-infrared light for illuminating tissue of interest;
capturing visible-light scene images and near-infrared scene images of the tissue of interest and outputting them in the form of a first video stream and a second video stream, respectively;
determining whether a current brightness value of the first video stream lies within a predefined first target brightness range and/or whether a current brightness value of the second video stream lies within a predefined second target brightness range;
if the current brightness value of the first video stream lies within the predefined first target brightness range and/or the current brightness value of the second video stream lies within the predefined second target brightness range, then
obtaining a brightness-fused image by fusing a current image frame in the first video stream with a current image frame in the second video stream based on brightness information of the current image frame in the first video stream and brightness information of the current image frame in the second video stream; and
obtaining a scene-fused image by fusing the current image frame in the first video stream with the brightness-fused image based on chroma information of the current image frame in the first video stream.

Optionally, if the current brightness value of the first video stream does not lie in the first target brightness range, a first exposure amount may be adjusted to cause the current brightness value of the first video stream to lie within the first target brightness range, wherein the first exposure amount is the product of a first exposure time and a first gain.

Additionally, if the current brightness value of the second video stream does not lie in the second target brightness range, a second exposure amount may be adjusted to cause the current brightness value of the second video stream to lie within the second target brightness range, wherein the second exposure amount is the product of a second exposure time and a second gain.

Optionally, when the current brightness value of the first video stream is below a lower limit of the first target brightness range, the current brightness value of the first video stream may be adjusted by increasing the first exposure amount.

Additionally, when the current brightness value of the second video stream is below a lower limit of the second target brightness range, the current brightness value of the second video stream may be adjusted by increasing the second exposure amount.

Optionally, adjusting the current brightness value of the first video stream by increasing the first exposure amount may comprise:

determining whether a maximum value of the first exposure time and a minimum value of the first gain satisfy an exposure amount requirement of the first target brightness range;
if the requirement is satisfied, with the first gain being kept at the minimum value, adjusting the first exposure time to cause the current brightness value of the first video stream to lie within the first target brightness range; and
if the requirement is not satisfied, determining whether the maximum value of the first exposure time and a maximum value of the first gain satisfy the exposure amount requirement of the first target brightness range and,
if so, with the first exposure time being kept at the maximum value, adjusting the first gain to cause the current brightness value of the first video stream to lie within the first target brightness range.

Additionally, adjusting the current brightness value of the second video stream by increasing the second exposure amount may comprise:

determining whether a maximum value of the second exposure time and a minimum value of the second gain satisfy an exposure amount requirement of the second target brightness range;

if the requirement is satisfied, with the second gain being kept at the minimum value, adjusting the second exposure time to cause the brightness value of the second video stream to lie within the second target brightness range; and

if the requirement is not satisfied, determining whether the maximum value of the second exposure time and a maximum value of the second gain satisfy the exposure amount requirement of the second target brightness range and,

if so, with the second exposure time being kept at the maximum value, adjusting the second gain to cause the brightness value of the second video stream to lie within the second target brightness range.

Optionally, when the current brightness value of the first video stream is above an upper limit of the first target brightness range, the current brightness value of the first video stream may be adjusted by reducing the first exposure amount.

Additionally, when the current brightness value of the second video stream is above an upper limit of the second target brightness range, the current brightness value of the second video stream may be adjusted by reducing the second exposure amount.

Optionally, adjusting the current brightness value of the first video stream by reducing the first exposure amount may comprise:

determining whether a maximum value of the first exposure time and a minimum value of the first gain satisfy an exposure amount requirement of the first target brightness range;

if the requirement is satisfied, with the first exposure time being kept at the maximum value, adjusting the first gain to cause the current brightness value of the first video stream to lie within the first target brightness range; and

if the requirement is not satisfied, determining whether a minimum value of the first exposure time and the minimum value of the first gain satisfy the exposure amount requirement of the first target brightness range and,

if so, with the first gain being kept at the minimum value, adjusting the first exposure time to cause the current brightness value of the first video stream to lie within the first target brightness range.

Additionally, adjusting the current brightness value of the second video stream by reducing the second exposure amount may comprise:

determining whether a maximum value of the second exposure time and a minimum value of the second gain satisfy an exposure amount requirement of the second target brightness range;

if the requirement is satisfied, with the second exposure time being kept at the maximum value, adjusting the second gain to cause the brightness value of the second video stream to lie within the second target brightness range; and

if the requirement is not satisfied, determining whether a minimum value of the second exposure time and the minimum value of the second gain satisfy the exposure amount requirement of the second target brightness range and,

if so, with the second gain being kept at the minimum value, adjusting the second exposure time to cause the brightness value of the second video stream to lie within the second target brightness range.

Optionally, when it is impossible to cause the current brightness value of the first video stream to lie within the first target brightness range by adjusting the first gain and the first exposure time, output power of a first light source module that provides the visible light may be adjusted to cause the current brightness value of the first video stream to lie within the first target brightness range.

Additionally, when it is impossible to cause the current brightness value of the second video stream to lie within the second target brightness range by adjusting the second gain and the second exposure time, output power of a third light source module that provides the near-infrared light may be adjusted to cause the current brightness value of the second video stream to lie within the second target brightness range.

Optionally, obtaining the brightness-fused image by fusing the current image frame in the first video stream with the current image frame in the second video stream based on the brightness information of the current image frame in the first video stream and the brightness information of the current image frame in the second video stream may comprise:

obtaining the brightness-fused image by fusing pixels of the current image frame in the first video stream with respective pixels of the current image frame in the second video stream based on the brightness information of the current image frame in the first video stream and the brightness information of the current image frame in the second video stream.

Optionally, obtaining the scene-fused image by fusing the current image frame in the first video stream with the brightness-fused image based on the chroma information of the current image frame in the first video stream may comprise:

assigning chroma information of pixels of the current image frame in the first video stream to respective pixels of the brightness-fused image as chroma values thereof.

Optionally, the method may further comprise, in case of the first video stream being output in a RAW or RGB format, before the current image frame in the first video stream is fused with the current image frame in the second video stream,

converting the current image frame in the first video stream into the YUV or YCbCr space.

Optionally, fusing the pixels of the current image frame in the first video stream with the respective pixels of the current image frame in the second video stream based on the brightness information of the current image frame in the first video stream and the brightness information of the current image frame in the second video stream may comprise:

deriving weights for brightness values of the pixels of the current image frame in the first video stream and weights for brightness values of the respective pixels of the current image frame in the second video stream from the brightness values of the pixels of the current image frame in the first video stream and the brightness values of the respective pixels of the current image frame in the second video stream, respectively, according to a predefined normal weight distribution as a function of brightness; and

obtaining brightness value of pixels of the brightness-fused image by weighting the brightness values of the pixels of the current image frame in the first video stream and the brightness values of the respective pixels of the current image frame in the second video stream with the derived weights for the brightness values of the pixels of the current image frame in the first video stream and weights for the brightness values of the respective pixels of the current image frame in the second video stream, respectively.

Optionally, the endoscope may be a three-dimensional (3D) endoscope, wherein:

capturing the visible-light scene images and near-infrared scene images of the tissue of interest and outputting them in the form of the first video stream and the second video stream comprises:

capturing first visible-light scene images, second visible-light scene images, first near-infrared scene images and second near-infrared scene images of the tissue of interest and outputting them in the form of a first visible-light video stream, a second visible-light video stream, a first near-infrared video stream and a second near-infrared video stream;

determining whether the current brightness value of the first video stream lies within the predefined first target brightness range comprises:

determining whether a current brightness value of the first visible-light video stream and/or a current brightness value of the second visible-light video stream lie(s) within the predefined first target brightness range;

determining whether the current brightness value of the second video stream lies within the predefined second target brightness range comprises:

determining whether a current brightness value of the first near-infrared video stream and/or a current brightness value of the second near-infrared video stream lie(s) within the predefined second target brightness range;

obtaining the brightness-fused image by fusing the current image frame in the first video stream with the current image frame in the second video stream based on the brightness information of the current image frame in the first video stream and the brightness information of the current image frame in the second video stream comprises:

obtaining a first brightness-fused image by fusing a current image frame in the first visible-light video stream with a current image frame in the first near-infrared video stream based on brightness information of the current image frame in the first visible-light video stream and brightness information of the current image frame in the first near-infrared video stream and obtaining a second brightness-fused image by fusing a current image frame in the second visible-light video stream with a current image frame in the second near-infrared video stream based on brightness information of the current image frame in the second visible-light video stream and brightness information of the current image frame in the second near-infrared video stream; and

obtaining the scene-fused image by fusing the current image frame in the first video stream with the brightness-fused image based on the chroma information of the current image frame in the first video stream comprises:

obtaining a first scene-fused image by fusing the current image frame in the first visible-light video stream with the first brightness-fused image based on chroma information of the current image frame in the first visible-light video stream and obtaining a second scene-fused image by fusing the current image frame in the second visible-light video stream with the second brightness-fused image based on chroma information of the current image frame in the second visible-light video stream.

To the above end, the present invention further provides a storage medium storing thereon a computer program, which, when executed by a processor, implements the method as defined above.

Compared with the prior art, the fluorescence endoscopy system, the control method and the storage medium proposed in the present invention have the advantages as follows: in the first mode, in addition to visible light, the illumination module can also provide excitation light, which, when irradiated on tissue of interest, can excite the tissue of interest to produce fluorescence, thereby helping an operator to obtain by observation information of the tissue that cannot be obtained under visible light illumination. In the second mode, the illumination module can provide both visible light and near-infrared light for illuminating tissue of interest, and the scene fusion module is configured to, when the current brightness value(s) of the first and/or second video stream(s) lie(s) within the respective target brightness range(s), the scene-fused image obtained by fusing the current visible-light and near-infrared image frames has a suitable brightness value, which allows rich details to be discerned. Therefore, on the one hand, the fluorescence endoscopy system of the present invention can effectively reduce image blur arising from camera movement, avoiding a user from making an erroneous determination of a lesion due to image blur and hence resulting in increased surgical accuracy and safety. In addition, an effectively increased signal-to-noise ratio can be achieved to allow more details to be discerned and obtained from the image. On the other hand, the fluorescence endoscopy system of the present invention is based on conventional hardware and can have various functions by making slight modifications thereto, which can meet various surgical needs of physicians and enable easy surgical operation.

## BRIEF DESCRIPTION OF THE DRAWINGS

Fig. 1 is a schematic diagram showing the structure of a fluorescence endoscopy system according to an embodiment of the present invention.
Fig. 2 schematically illustrates control of a fluorescence endoscopy system for mode switching according to an embodiment of the present invention.
Fig. 3 schematically illustrates operation of a beam-splitting prism group according to an embodiment of the present invention.
Fig. 4 is a schematic spectral diagram of a visible-light band-pass filter according to an embodiment of the present invention.
Fig. 5 is a schematic spectral diagram of a near-infrared band-pass filter according to an embodiment of the present invention.
Fig. 6 is a schematic flowchart of a process of tuning a current brightness value B1 of a first video stream into a first target brightness range (B1$_{min}$, B1$_{max}$) through adjusting a first exposure amount in a second mode according to an embodiment of the present invention.
Fig. 7 schematically illustrates a normal weight distribution as a function of brightness according to an embodiment of the present invention.
Fig. 8 schematically illustrates image fusion in a second mode according to an embodiment of the present invention.
Fig. 9 schematically illustrates a flowchart of a process for controlling a fluorescence endoscopy system in a second mode according to an embodiment of the present invention.

In these figures,
100 denotes an endoscope; 200, an illumination module; 300, an endoscope drive module; 400, an endoscope control module; 500, a scene fusion module ; 600, tissue of interest; 111, a visible-light image sensor; 111A, a first visible-light image sensor; 111B, a second visible-light image sensor; 121, a near-infrared image sensor; 121A, a first near-infrared image sensor; 121B, a second near-infrared image sensor; 410, a first control unit; 310, a first drive unit; 311, a first visible-light drive unit; 312, a second visible-light drive unit; 320, a second drive unit; 321, a first near-infrared drive unit; 322, a second near-infrared drive unit; 411, a first brightness acquisition means; 412, a first exposure control means; 421, a second brightness acquisition means; 422, a second exposure control means; 413, a first illumination adjustment means; 423, a second illumination adjustment means; 130, a beam-splitting prism group; 131, a visible-light band-pass filter; 132, a near-infrared band-pass filter; 133, a first beam-splitting prism; 134,a second beam-splitting prism; 510, a first image fusion unit; 520, a second image fusion unit; 530, an image mode switching unit; 210, a light source unit; 220, an illumination controller; 211, a first light source module; 212, a second light source module; 213, a third light source module; 221, a mode switching unit; 222, a power control unit; 700, a video pipeline; 800, a central controller; 810, a video overlay unit; 820, a user input device; 830, a user interface; and 900, a monitor.

## DETAILED DESCRIPTION

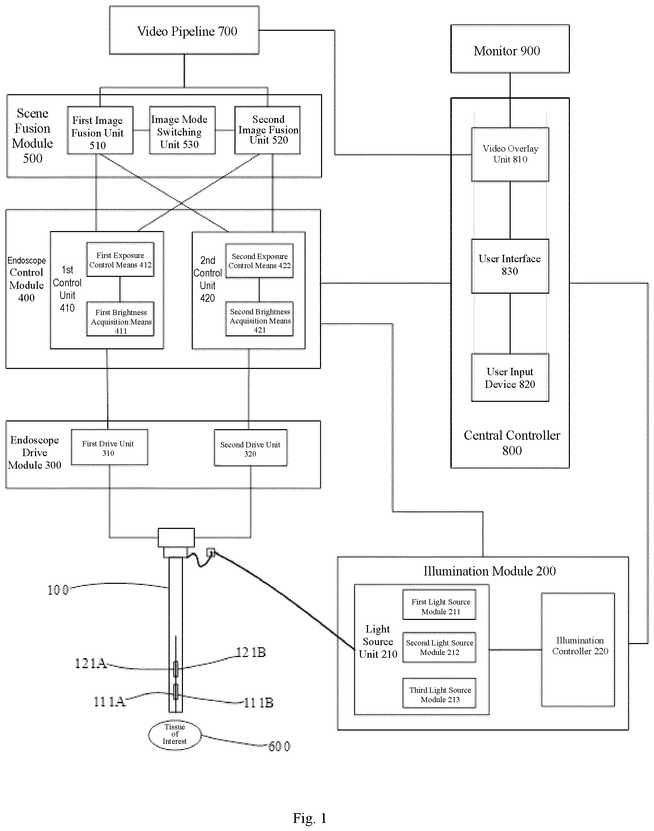

The fluorescence endoscopy system, control method and storage medium proposed in the present invention will be described in greater detail below with reference to Figs. 1 to 9 and to specific embodiments. Advantages and features of the invention will become more apparent from the following description. Note that the figures are provided in a very simplified form not necessarily drawn to exact scale and for the only purpose of facilitating easy and clear description of the disclosed embodiments. In order that objects, features and advantages of the present invention may become readily apparent, reference is made to the accompanying drawings. It should be noted that architectural, proportional, dimensional and other details in the figures are presented only for the purpose of facilitating, in conjunction with the disclosure herein, the understanding and reading of those familiar with the art rather than being intended to limit conditions under which the present invention can be implemented. Therefore, they are technically of no substantive significance, and any and all architectural modifications, proportional variations or dimensional changes that do not affect the benefits and objects of the present invention are considered to fall within the scope of the teachings herein.

It is to be noted that, as used herein, relational terms such as first and second, etc., are only used to distinguish one entity or operation from another entity or operation, and do not necessarily require or imply these entities having such an order or sequence. Moreover, the terms "include," "including," or any other variations thereof are intended to cover a non-exclusive inclusion within a process, method, article, or apparatus that comprises a list of elements including not only those elements but also those that are not explicitly listed, or other elements that are inherent to such processes, methods, goods, or equipment. In the case of no more limitation, the element defined by the sentence "includes a ..." does not exclude the existence of another identical element in the process, the method, or the device including the element.

In principle, the present invention seeks to provide a fluorescence endoscopy system, a control method and a storage medium capable of imaging particular tissue with fluorescence and obtaining information that cannot be obtained with visible light. Moreover, they circumvent the problems of smear and aggravated image noise that may arise from the use of a conventional method of fusion of successive frames of captured images for addressing obscured details in the images in the event of bleeding occurring in a surgical procedure performed in an environment illuminated with visible light.

To this end, the present invention provides a fluorescence endoscopy system. Reference is now made to Fig. 1, a schematic diagram showing the structure of a fluorescence endoscopy system according to an embodiment of the present invention. As shown in Fig. 1, the fluorescence endoscopy system includes an endoscope 100, an illumination module 200, an endoscope drive module 300 and a scene fusion module 500. Operating modes of the fluorescence endoscopy system include a first mode (i.e., fluorescence mode) and a second mode (i.e., bleeding mode).

The illumination module 200 is configured to provide visible light, excitation light and near-infrared light. The visible light and the near-infrared light are configured to illuminate, and be reflected by, tissue of interest 600, and the excitation light is configured to excite the tissue of interest to produce fluorescence. The present invention is not limited to any particular location of the illumination module 200. For example, output light from the illumination module 200 may be transmitted to an end of the endoscope 100 through a connector received in an illuminate channel of the endoscope 100 and then reach the tissue of interest 600. The connector may be, for example, an optical fiber.

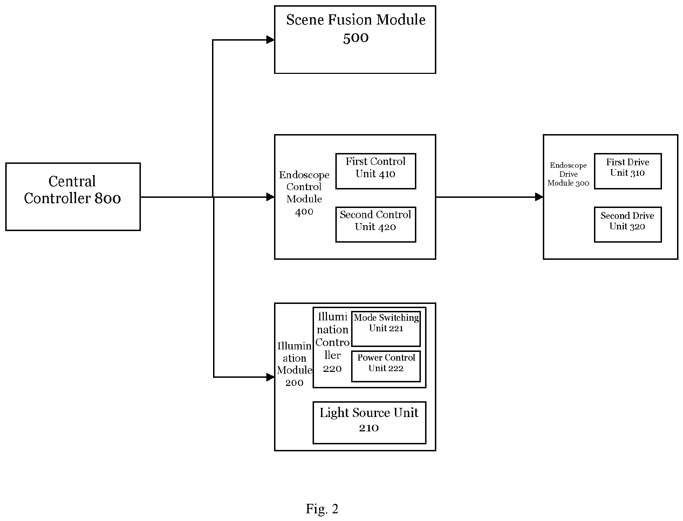

In the fluorescence mode of the fluorescence endoscopy system, the illumination module 200 emits visible light and excitation light to the tissue of interest 600, which then reflects the visible light and is excited by the excitation light to emit fluorescence. In the bleeding mode of the fluorescence endoscopy system, the illumination module 200 emits visible light and near-infrared light to the tissue of interest 600. Likewise, the tissue of interest 600 reflects both the visible and near-infrared light. In this embodiment, a spectrum of the excitation light ranges from 803 nm to 812 nm, and a spectrum of the fluorescence emitted in response to the excitation ranges from 830 nm to 840 nm. Moreover, a spectrum of the near-infrared light ranges from 935 nm to 945 nm. Reference is now made to Figs. 1 and 2. Fig. 2 schematically illustrates control of the fluorescence endoscopy system for mode switching according to an embodiment of the present invention. As shown in Figs. 1 and 2, the illumination module 200 includes a light source unit 210 and an illumination controller 220. The light source unit 210 includes a first light source module 211 for providing visible light, a second light source module 212 for providing excitation light and a third light source module 213 for providing near-infrared light. The illumination controller 220 includes a mode switching unit 221 for switching the system between the fluorescence and bleeding modes and a power control unit 222 for controlling output power of the light source unit 210. The mode switching unit 221 is coupled to the central controller 800 described below, thus enabling an operator to control the mode switching unit 221 using the central controller 800 to switch the system between the fluorescence and bleeding modes. In the fluorescence mode of the fluorescence endoscopy system, the first light source module 211 and the second light source module 212 are activated under the control of the illumination controller 220. In the bleeding mode of the fluorescence endoscope, the first light source module 211 and the third light source module 213 are activated under the control of the illumination controller 220.

As shown in Fig. 1, the endoscope 100 includes a visible-light image sensor 111 and a near-infrared image sensor 121. The visible-light image sensor 111 is configured to capture the reflected visible light, obtain visible-light scene images of the tissue of interest 600 and output them in the form of a first video stream. In this embodiment, both the fluorescence produced by the excited tissue of interest and the near-infrared light produced by the illumination module 200 are in the near-infrared spectrum. Therefore, in this embodiment, the fluorescence produced by the excited tissue of interest and the near-infrared light reflected from the tissue of interest are captured by the near-infrared image sensor 121. That is, in the fluorescence mode of the fluorescence endoscopy system, the near-infrared image sensor 121 is configured to capture the fluorescence that carries scene information of the tissue of interest 600, obtain fluorescence scene images of the tissue of interest 600 by performing optical-to-electronic conversion on the scene information of the tissue of interest 600 and output them in the form of a second video stream. In the bleeding mode of the fluorescence endoscopy system, the near-infrared image sensor 121 is configured to capture the reflected near-infrared light that carries scene information of the tissue of interest 600, obtain near-infrared scene images of the tissue of interest 600 by performing optical-to-electronic conversion on the scene information of the tissue of interest 600 and output them in the form of a second video stream.

Each of the visible-light image sensor 111 and the near-infrared image sensor 121 may be implemented as a complementary metal oxide semiconductor (CMOS) or charge coupled device (CCD) sensor.

The endoscope 100 further includes a beam-splitting prism group 130 configured to separate the visible light reflected from the tissue of interest 600 from the near-infrared light reflected from the tissue of interest 600 in the bleeding mode. In the fluorescence mode, it is also configured to separate the visible light reflected from the tissue of interest 600 from the fluorescence produced in response to the excitation. As such, the reflected visible light can be captured by a photosensitive surface of the visible-light image sensor 111, and the reflected near-infrared light or the fluorescence can be captured by a photosensitive surface of the near-infrared image sensor 121.

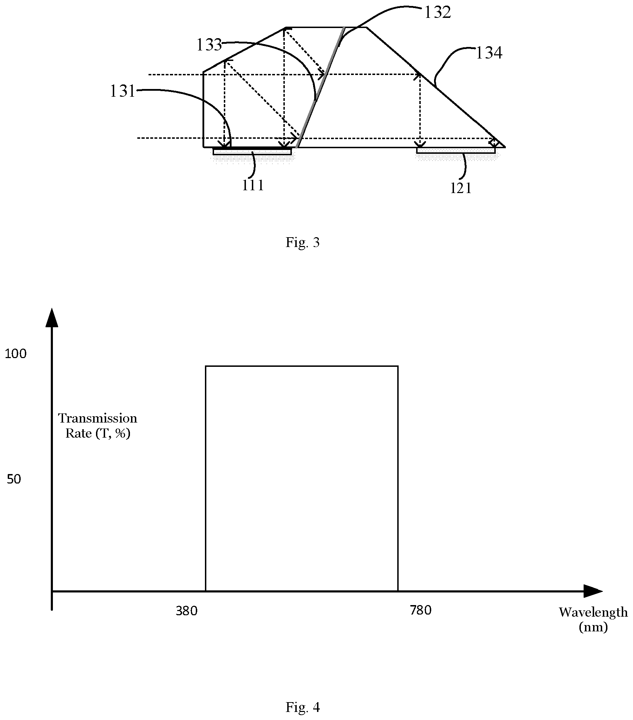

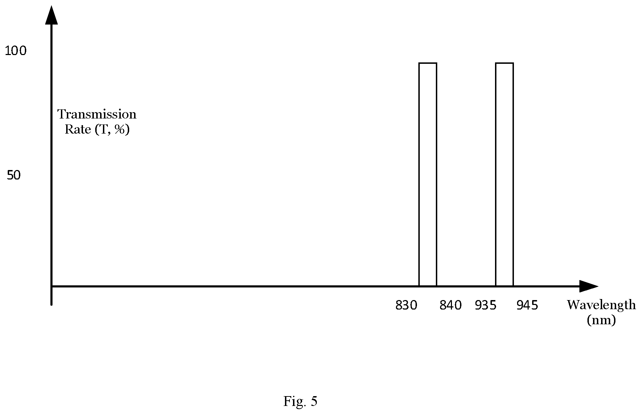

Reference is now made to Figs. 3 to 5. Fig. 3 schematically illustrates operation of the beam-splitting prism group according to an embodiment of the present invention. Fig. 4 is a schematic spectral diagram of a visible-light band-pass filter according to an embodiment of the present invention. Fig. 5 is a schematic spectral diagram of a near-infrared band-pass filter according to an embodiment of the present invention. Preferably, as shown in Fig. 3, the beam-splitting prism group 130 includes a first beam-splitting prism 133, a second beam-splitting prism 134, a visible-light band-pass filter 131 and a near-infrared band-pass filter 132. The visible-light band-pass filter 131 is configured to allow passage of visible light therethrough and block light at other wavelengths. The near-infrared band-pass filter 132 is configured to allow passage of near-infrared light therethrough and block light at other wavelengths. The near-infrared band-pass filter 132 is disposed between the first beam-splitting prism 133 and the second beam-splitting prism 134. On a surface of the first beam-splitting prism 133 adjacent to the second beam-splitting prism 134, a semi-transmissive, semi-reflective film which reflects part of light incident thereon and allows transmission of the remainder thereof is provided. The photosensitive surface of the visible-light image sensor 111 is adjacent to an exit surface of the first beam-splitting prism 133, and the visible-light band-pass filter 131 is disposed between the exit surface of the first beam-splitting prism 133 and the photosensitive surface of the visible-light image sensor 111. Similarly, the photosensitive surface of the near-infrared image sensor 121 is adjacent to an exit surface of the second beam-splitting prism 134. Here, the "exit surface of the first beam-splitting prism 133" refers to a surface of the first beam-splitting prism 133 where reflected light leaves the first beam-splitting prism 133, and the "exit surface of the second beam-splitting prism 134" refers to a surface of the second beam-splitting prism 134 where light that has transmitted therethrough leaves the second beam-splitting prism 134. As shown in Fig. 3, the photosensitive surface of the visible-light image sensor 111 and the photosensitive surface of the near-infrared image sensor 121 are parallel to an optical axis. This arrangement allows the sizes of the image sensors to be less limited by the size of the endoscope, resulting in improved image quality. When the mixed light (consisting of the visible light reflected from the tissue of interest 600 and the fluorescence produced by the excited tissue of interest 600 in the fluorescence mode, or of the visible light and the near-infrared light both reflected from the tissue of interest 600 in the bleeding mode) travels along the optical axis and enters the first beam-splitting prism 133, part of it will be reflected, and the remainder will transmit through the first beam-splitting prism 133. The reflected part of the mixed light is again reflected (e.g., the primary reflection shown in Fig. 3) and leaves the first beam-splitting prism 133 through the exit surface thereof. Subsequently, it is incident on the visible-light band-pass filter 131, and a component thereof in the spectrum from 380 nm to 780 nm (i.e., a visible light component) passes through the filter and is captured by the visible-light image sensor 111. The part of the mixed light that has transmitted through the first beam-splitting prism 133 is incident on the near-infrared band-pass filter 132, and components thereof in the spectra respectively from 830 nm to 840 nm and from 935 nm to 945 nm pass through the filter and enter the second beam-splitting prism 134 along the optical axis. These components are reflected (e.g., the primary reflection shown in Fig. 3), leave the second beam-splitting prism 134 through the exit surface of the second beam-splitting prism 134 and are captured by the near-infrared image sensor 121. As shown in Figs. 4 and 5, in this embodiment, as the visible-light band-pass filter 131 allows passage of light in the spectrum from 380 nm to 780 nm, it can be ensured that the reflected light of the visible light emanated from the illumination module 200 enters the visible-light image sensor 111. Moreover, since the near-infrared band-pass filter 132 allows passage of light in the spectra respectively from 830 nm to 840 nm and from 935 nm to 945 nm therethrough, it can be ensured that the reflected light of the near-infrared light emanated from the illumination module 200 and the fluorescence enter the near-infrared image sensor 121. In an alternative embodiment, the endoscope is a three-dimensional (3D) endoscope, and the fluorescence endoscopy system is accordingly a 3D endoscopy system. In this case, the visible-light scene images may include first visible-light scene images and second visible-light scene images, and the near-infrared scene images may include first near-infrared scene images and second near-infrared scene images. Moreover, two beam-splitting prism groups 130 may be included. Accordingly, two visible-light image sensors may be included: a first visible-light image sensor 111A and a second visible-light image sensor 111B. The first visible-light image sensor 111A may be configured to capture the first visible-light scene images of the tissue of interest and output them in the form of a first visible-light video stream, and the second visible-light image sensor 111B may be configured to capture the second visible-light scene images of the tissue of interest and output them in the form of a second visible-light video stream. Further, two near-infrared image sensors may be included and they are a first near-infrared image sensor 121A and a second near-infrared image sensor 121B. In the fluorescence mode, the first near-infrared image sensor 121A may be configured to capture first fluorescence scene images and output them in the form of a first fluorescence video stream, and the second near-infrared image sensor 121B may be configured to capture second fluorescence scene images of the tissue of interest and output them in the form of a second fluorescence video stream. In the bleeding mode, the first near-infrared image sensor 121A may be configured to capture first near-infrared scene images of the tissue of interest and output them in the form of a first near-infrared video stream, and the second near-infrared image sensor 121B may be configured to capture second near-infrared scene images of the tissue of interest and output them in the form of a second near-infrared video stream. It is to be stressed that the terms "first" and "second" used in this embodiment to refer to the elements do not imply any order of priority. For example, the first visible-light scene images may be either left visible-light scene images or right visible-light scene images for the endoscope.

The endoscope drive module 300 includes a first drive unit 310 and a second drive unit 320. In the bleeding mode, the first drive unit 310 is configured to drive the visible-light image sensor 111 to capture the visible-light scene images based on a first exposure time $T_1$ and a first gain Gi, and the second drive unit 320 is configured to drive the near-infrared image sensor 121 to capture the near-infrared scene images based on a second exposure time $T_2$ and a second gain $G_2$. Preferably, in the fluorescence mode, the first drive unit 310 is configured to drive the visible-light image sensor 111 to capture the visible-light scene images based on a third exposure time $T_3$ and a third gain $G_3$, and the second drive unit 320 is configured to drive the near-infrared image sensor 121 to capture the fluorescence scene images based on a fourth exposure time $T_4$ and a fourth gain $G_4$.

Preferably, the fluorescence endoscopy system further includes an endoscope control module 400 communicatively connected to the endoscope drive module 300. The endoscope control module 400 includes a first control unit 410 and/or a second control unit 420. The first control unit 410 is configured to maintain a current brightness value $B_1$ of the first video stream in a predefined first target brightness range ($B_{1min}$, $B_{1max}$) in the bleeding mode, and the second control unit 420 is configured to maintain a current brightness value $B_2$ of the second video stream in a predefined second target brightness range ($B_{2mm}$, $B_{2max}$) in the bleeding mode. In the bleeding mode of the fluorescence endoscopy system, the fluorescence endoscopy system is desired to maintain the brightness value of the first video stream and/or the brightness value of the second video stream within the desired brightness range(s), as required by image fusion and other subsequent processing. Accordingly, in the bleeding mode, the first control unit 410 and/or the second control unit 420 may determine whether the current brightness value $B_1$ of the first video stream lies within the first target brightness range ($B_{1min}$, $B_{1max}$) and/or whether the current brightness value $B_2$ of the second video stream lies within the second target brightness range ($B_{2min}$, $B_{2max}$). If not, an exposure amount of each image sensor that produces the insufficiently or excessively bright video stream (which is assessed with the product of the corresponding exposure time T and gain G) is tuned to adjust the current brightness value. In this embodiment, the fluorescence endoscope has a constant aperture size, and therefore, the exposure amount may be assessed with the product of the exposure time T and the gain G. In other embodiments, the fluorescence endoscope may have a varying aperture size, and the exposure amount may be accordingly assessed with the product of the aperture size, the exposure time T and the gain G. That is, when the determination is negative, the first control unit 410 tunes a first exposure amount of the visible-light image sensor 111 (here, assessed with the product of the first exposure time $T_1$ and the first gain $G_1$), thereby bringing the current brightness value $B_1$ of the first video stream into the predefined first target brightness range ($B_{1min}$, $B_{1max}$), and/or the second control unit 420 adjusts a second exposure amount of the near-infrared image sensor 121 (here, assessed with the product of the second exposure time $T_2$ and the second gain $G_2$), thereby bringing the current brightness value $B_2$ of the second video stream into the predefined second target brightness range ($B_{2min}$, $B_{2max}$). In this embodiment, the "current brightness value" of each video stream refers to a brightness value of a current image frame in the video stream. Accordingly, the current brightness value $B_1$ of the first video stream refers to a brightness value of a current image frame in the first video stream, and the current brightness value $B_2$ of the second video stream refers to a brightness value of a current image frame in the second video stream. In the bleeding mode of the fluorescence endoscopy system, the first drive unit 310 receives parameters from the first control unit 410 including the first exposure time $T_1$ and the first gain $G_1$ to enable the visible-light scene images captured by the visible-light image sensor 111 to satisfy the aforementioned brightness value requirement, and the second drive unit 320 receives parameters from the second control unit 420 including the second exposure time $T_2$ and the second gain $G_2$ to enable the near-infrared scene images captured by the near-infrared image sensor 121 to satisfy the aforementioned brightness value requirement. Preferably, the first control unit 410 is also configured to maintain the current brightness value $B_1$ of the first video stream within a predefined third target brightness range ($B_{3min}$, $B_{3max}$) in the fluorescence mode, and/or the second control unit 420 is configured to maintain the current brightness value $B_2$ of the second video stream in a predefined fourth target brightness range ($B_{4min}$, $B_{4max}$) in the fluorescence mode.

In one embodiment, the fluorescence endoscopy system adjusts the current brightness values of the video streams only in the bleeding mode. In this case, the first target brightness range ($B_{1min}$, $B_{1max}$) and the second target brightness range ($B_{2min}$, $B_{2max}$) may be configured in the first control unit 410 and the second control unit 420, respectively. In response to the endoscope control module 400 receiving a command for activating the bleeding mode from the central controller 800 (detailed below), the first control unit 410 and the second control unit 420 adjust the current brightness values of the first and second video streams according to the first target brightness range ($B_{1min}$, $B_{1max}$) and the second target brightness range ($B_{2min}$, $B_{2max}$), respectively. In another embodiment, the fluorescence endoscopy system adjusts the current brightness values of the video streams in both the bleeding and fluorescence modes. In this case, the first control unit 410 and the second control unit 420 may be always kept active. Moreover, the first target brightness range ($B_{1min}$, $B_{1max}$) and the third target brightness range ($B_{3min}$, $B_{3max}$) may be both configured in the first control unit 410, and the second target brightness range ($B_{2min}$, $B_{2max}$) and the fourth target brightness range ($B_{4min}$, $B_{4max}$) may be both configured in the second control unit 420. Upon receiving a command for activating the bleeding mode, the central controller 800 may control the first control unit 410 and the second control unit 420 to adjust the current brightness values of the first and second video streams according to the first target brightness range ($B_{1min}$, $B_{1max}$) and the second target brightness range ($B_{2min}$, $B_{2max}$). Upon receiving a command for activating the fluorescence mode, the central controller 800 may control the first control unit 410 and the second control unit 420 to adjust the current brightness values of the first and second video streams according to the third target brightness range ($B_{3min}$, $B_{3max}$) and the fourth target brightness range ($B_{4min}$, $B_{4max}$). Alternatively, the first target brightness range ($B_{1min}$, $B_{1max}$), the second target brightness range ($B_{2mm}$, $B_{2max}$), the third target brightness range ($B_{3min}$, $B_{3max}$) and the fourth target brightness range ($B_{4min}$, $B_{4max}$) may be transmitted to the endoscope control module 400 during operation of the central controller 800 for switching the endoscopy system from one mode to another.

Preferably, the first control unit 410 includes a first brightness acquisition means 411 and a first exposure control means 412. The first brightness acquisition means 411 is coupled to the visible-light image sensor 111, and the first exposure control means 412 is coupled to the first brightness acquisition means 411 and the first drive unit 310. The first brightness acquisition means 411 is configured to receive the first video stream from the visible-light image sensor 111, acquire the brightness value of the current image frame in the first video stream in real time, i.e., the current brightness value $B_1$ of the first video stream, and provide the current brightness value $B_1$ to the first exposure control means 412. The first exposure control means 412 is configured to determine whether the received current brightness value $B_1$ of the first video stream lies within the predefined first target brightness range ($B_{1min}$, $B_{1max}$). If the current brightness value $B_1$ of the received first video stream is not within the predefined first target brightness range ($B_{1min}$, $B_{1max}$), the first exposure amount of the visible-light image sensor 111 is adjusted and output to the first drive unit 310 so that the current brightness value $B_1$ of the first video stream shifts into the predefined first target brightness range ($B_{1min}$, $B_{1max}$). Those skilled in the art would appreciate that the current frame in the first video stream and hence the image of the current frame therein change dynamically. Thus, by the phrase "so that the current brightness value $B_1$ of the first video stream shifts into the predefined first target brightness range", it is intended to mean that a visible-light scene image newly captured by the visible-light image sensor 111 is adjusted based on the first exposure time $T_1$ and the first gain $G_1$ derived from the current image frame so that when the subsequent frame (i.e., the newly captured visible-light scene image) becomes a current frame, a brightness value $B_1$ of the current image frame lies in the predefined first target brightness range ($B_{1min}$, $B_{1max}$).

Similarly, the second control unit 420 includes a second brightness acquisition means 421 and a second exposure control means 422. The second brightness acquisition means 421 is coupled to the near-infrared image sensor 121, and the second exposure control means 422 is coupled to the second brightness acquisition means 421 and the second drive unit 320. The second brightness acquisition means 421 is configured to receive the second video stream from the near-infrared image sensor 121, acquire the brightness value of the current image frame in the second video stream in real time, i.e., the current brightness value $B_2$ of the second video stream, and provide the current brightness value $B_2$ to the second exposure control means 422. The second exposure control means 422 is configured to determine whether the received current brightness value $B_2$ of the second video stream lies within the predefined second target brightness range ($B_{2min}$, $B_{2max}$). If the current brightness value $B_2$ of the received second video stream is not within the predefined second target brightness range ($B_{2min}$, $B_{2max}$), the second exposure amount of the near-infrared image sensor 121 is adjusted and output to the second drive unit 320 so that the current brightness value $B_2$ of the second video stream shifts into the predefined second target brightness range ($B_{2min}$, $B_{2max}$). Likewise,

those skilled in the art would appreciate that the current frame in the second video stream and hence the image of the current frame therein change dynamically. Thus, by the phrase "so that the current brightness value $B_2$ of the second video stream shifts into the predefined second target brightness range", it is intended to mean that a near-infrared scene image newly captured by the near-infrared image sensor 121 is adjusted based on the second exposure time $T_2$ and the second gain $G_2$ derived from the current image frame so that when the subsequent frame (i.e., the newly captured near-infrared scene image) becomes a current frame, a brightness value $B_2$ of the current image frame lies in the predefined second target brightness range $(B_{2mm}, B_{2max})$.

The present invention is not limited to any particular method of acquiring the current brightness value $B_1$ of the first video stream. For example, when the first video stream is YUV or $YC_bC_r$-coded, the first brightness acquisition means 411 may take an averaged or weighted value of Y values of all or some pixels in the current image frame of the first video stream as the current brightness value $B_1$. As another example, when the first video stream output from the visible-light image sensor 111 is RAW- or RGB-coded, the first brightness acquisition means 411 may derive brightness values of pixels in the current image frame of the first video stream from their RGB values and then take an averaged or weighted value of the brightness values of all or some of the pixels in the current image frame of the first video stream as the current brightness value $B_1$. The derivation of the brightness values Y of the pixels from their RGB values may be accomplished by simply taking the G values that are more sensitive to the human eye, or values obtained by weighting the R, G and B values, for example, according to $Y=0.2126*R + 0.7152*G + 0.0722*B$ or $Y=0.299*R + 0.587*G + 0.114*B$, as the brightness values. The current brightness value $B_2$ of the second video stream may be acquired in a similar manner as the acquisition of the current brightness value $B_1$ of the first video stream, or using an approach that takes advantage of the nature of the near-infrared images, which is, however, not described in further detail herein for the sake of brevity.

Preferably, the first control unit 410 further includes a first illumination adjustment means 413, which is communicatively connected to the first exposure control means 412 and configured to control, when the first gain $G_1$ and the first exposure time $T_1$ output from the first exposure control means 412 fail to maintain the current brightness value $B_1$ of the first video stream within the first target brightness range $(B_{1min}, B_{1max})$, the illumination module 200 to adjust output power of the first light source module 211 (i.e., a luminous flux of visible light) so that the current brightness value $B_1$ of the first video stream is brought into the first target brightness range $(B_{1min}, B_{1max})$. Additionally, the second control unit 420 further includes a second illumination adjustment means 423, which is communicatively connected to the second exposure control means 422 and configured to control, when the second gain $G_2$ and the second exposure time $T_2$ output from the second exposure control means 422 fail to maintain the current brightness value $B_2$ of the second video stream within the second target brightness range $(B_{2mm}, B_{2max})$, the illumination module 200 to adjust output power of the third light source module 213 (i.e., a luminous flux of near-infrared light) so that the current brightness value $B_2$ of the second video stream is brought into the second target brightness range $(B_{2min}, B_{2max})$.

Specifically, when receiving the current brightness value $B_1$ of the first video stream from the first brightness acquisition means 411, the first exposure control means 412 determines whether the current brightness value $B_1$ is within the predefined first target brightness range $(B_{1min}, B_{1max})$. If the current brightness value $B_1$ lies within the predefined first target brightness range $(B_{1min}, B_{1max})$, the first exposure time $T_1$ and the first gain $G_1$ are output to the first drive unit 310, or no data exchange occurs between the first exposure control means 412 and the first drive unit 310. If the current brightness value $B_1$ is outside of the predefined first target brightness range $(B_{1min}, B_{1max})$, the first exposure amount of the visible-light image sensor 111 is adjusted to bring the current brightness value $B_1$ into the predefined first target brightness range $(B_{1min}, B_{1max})$. If it is impossible to shift the current brightness value $B_1$ into the predefined target brightness range $(B_{1min}, B_{1max})$ through adjusting the first exposure time $T_1$ and the first gain $G_1$, then the first exposure control means 412 feeds the information back to the first illumination adjustment means 413 connected to the illumination module 200. In response, the output power of the first light source module 211 (i.e., the luminous flux of visible light) is adjusted to tune the current brightness value $B_1$ of the first video stream into the first target brightness range $(B_{1min}, B_{1max})$.

Likewise, when receiving the current brightness value $B_2$ of the second video stream from the second brightness acquisition means 421, the second exposure control means 422 determines whether the current brightness value $B_2$ is within the predefined second target brightness range $(B_{2min}, B_{2max})$. If the current brightness value $B_2$ lies within the predefined second target brightness range $(B_{2mm}, B_{2max})$, the second exposure time $T_2$ and the second gain $G_2$ are output to the second drive unit 320, or no data exchange occurs between the second exposure control means 422 and the second drive unit 320. If the current brightness value $B_2$ is outside of the predefined second target brightness range $(B_{2min}, B_{2max})$, the second exposure amount of the near-infrared image sensor 121 is adjusted to bring the current brightness value $B_2$ into the predefined second target brightness range $(B_{2min}, B_{2max})$. If it is impossible to shift the current brightness value $B_2$ into the predefined target brightness range $(B_{2min}, B_{2max})$ through adjusting the second exposure time $T_2$ and the second gain $G_2$, then the second exposure control means 422 feeds the information back to the second illumination adjustment means 423 connected to the illumination module 200. In response, the output power of the third light source module 213 (i.e., the luminous flux of near-infrared light) is adjusted to tune the current brightness value $B_2$ of the second video stream into the second target brightness range ($B_{2mm}$, $B_{2max}$).

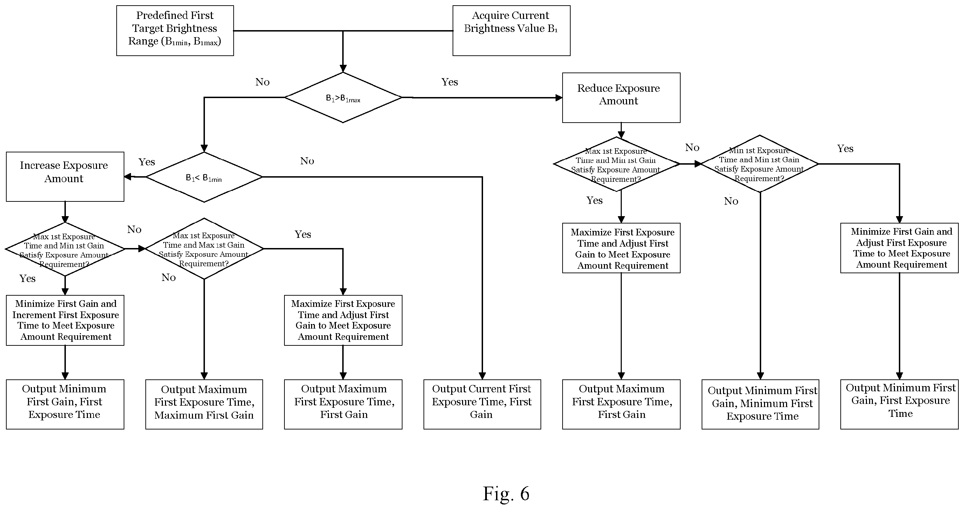

Fig. 6 is a schematic flowchart of a process of tuning the current brightness value $B_1$ of the first video stream into the first target brightness range ($B_{1min}$, $B_{1max}$) through adjusting the first exposure amount according to an embodiment of the present invention. As shown in Fig. 6, the process includes the steps as follows.

On the one hand, when the current brightness value $B_1$ of the first video stream is below the lower limit $B_{1min}$ of the first target brightness range, the first exposure control means 412 increases the first exposure amount of the visible-light image sensor 111 to adjust the current brightness value $B_1$ of the first video stream. First of all, the first exposure time $T_1$ is increased, and it is determined whether a maximum value $T_{1max}$ of the first exposure time and a minimum value $G_{1min}$ of the first gain satisfy an exposure amount requirement of the first target brightness range ($B_{1min}$, $B_{1max}$).

If the requirement is satisfied, with the first gain being kept at the minimum value $G_{1min}$, the first exposure time $T_1$ is adjusted so that the current brightness value $B_1$ of the first video stream shifts into the first target brightness range ($B_{1min}$, $B_{1max}$), and the minimum value $G_{1min}$ of the first gain and the adjusted first exposure time $T_1$ are output to the first drive unit 310.

If the requirement is not satisfied, the first gain $G_1$ is increased, and it is determined whether the maximum value $T_{1max}$ of the first exposure time and a maximum value $G_{1max}$ of the first gain satisfy the exposure amount requirement of the first target brightness range ($B_{1min}$, $B_{1max}$). If the requirement is still not satisfied, then the current values of the first exposure time $T_1$ and the first gain $G_1$ are maintained, and the first exposure control means 412 feeds the information indicating that the current brightness value $B_1$ remains outside of the first target brightness range ($B_{1min}$, $B_{1max}$) (e.g., the information may indicate that the first exposure time $T_1$ is at the maximum value $T_{1max}$ and the first gain $G_1$ at the maximum value $G_{1max}$) to the first illumination adjustment means 413. If the requirement is satisfied, then with the first exposure time being kept at the maximum value $T_{1max}$, the first gain $G_1$ is adjusted to tune the current brightness value $B_1$ of the first video stream into the first target brightness range ($B_{1min}$, $B_{1max}$), and the maximum value $T_{1max}$ of the first exposure time and the adjusted first gain $G_1$ are output to the first drive unit 310.

As an example, with the first gain being kept at the minimum value $G_{1min}$, the first exposure time $T_1$ may be adjusted by calculating $T_{1c}=T_1' \times G_1'/G_{1min}$ from a first exposure amount ($T_1' \times G_1'$) of the previous frame and incrementing the first exposure time $T_1$ from $T_{1c}$ until the current brightness value $B_1$ of the first video stream shifts into the first target brightness range ($B_{1min}$, $B_{1max}$). As an example, with the first exposure time being kept at the maximum value $T_{1max}$, the first gain $G_1$ may be adjusted by calculating $G_{1c}=T_1' \times G_1'/T_{1max}$ from a first exposure amount ($T_1' \times G_1'$) of the previous frame and incrementing the first gain $G_1$ from $G_{1c}$ until the current brightness value $B_1$ of the first video stream shifts into the first target brightness range ($B_{1min}$, $B_{1max}$).

On the other hand, when the current brightness value $B_1$ of the first video stream is above the upper limit $B_{1max}$ of the first target brightness range ($B_{1min}$, $B_{1max}$), the first exposure control means 412 reduces the first exposure amount of the visible-light image sensor 111 to adjust the current brightness value $B_1$ of the first video stream. Specifically, first of all, the first gain $G_1$ is reduced, and it is determined whether the maximum value $T_{1max}$ of the first exposure time and the minimum value $G_{1min}$ of the first gain satisfy an exposure amount requirement of the first target brightness range ($B_{1min}$, $B_{1max}$). If the requirement is satisfied, with the first exposure time being kept at the maximum value $T_{1max}$, the first gain $G_1$ is adjusted so that the current brightness value $B_1$ of the first video stream shifts into the first target brightness range ($B_{1min}$, $B_{1max}$), and the maximum value $T_{1max}$ of the first exposure time and the adjusted first gain $G_1$ are output to the first drive unit 310.

If the requirement is not satisfied, the first exposure time $T_1$ is reduced, and it is determined whether a minimum value $T_{1min}$ of the first exposure time and the minimum value $G_{1min}$ of the first gain satisfy the exposure amount requirement of the first target brightness range ($B_{1min}$, $B_{1max}$). If the requirement is still not satisfied, then the current values of the first exposure time $T_1$ and the first gain $G_1$ are maintained, and the first exposure control means 412 feeds the information indicating that the current brightness value $B_1$ remains outside of the first target brightness range ($B_{1min}$, $B_{1max}$) (e.g., the information may indicate that the first exposure time $T_1$ is at the minimum value $T_{1min}$ and the first gain $G_1$ at the minimum value $G_{1min}$) to the first illumination adjustment means 413 that is connected to the illumination module 200. If the requirement is satisfied, then with the first gain being kept at the minimum value $G_{1min}$, the first exposure time $T_1$ is adjusted to tune the current brightness value $B_1$ of the first video stream into the first target brightness range ($B_{1min}$, $B_{1max}$), and the minimum value $G_{1min}$ of the first gain and the adjusted first exposure time $T_1$ are output to the first drive unit 310.

As an example, with the first exposure time being kept at the maximum value $T_{1max}$, the first gain $G_1$ may be adjusted by calculating $G_{1c}=T_1' \times G_1'/T_{1max}$ from a first exposure amount ($T_1' \times G_1'$) of the previous frame and decrementing the first gain $G_1$ from $G_{1c}$ until the current brightness value $B_1$ of the first video stream shifts into the first target brightness range ($B_{1min}$, $B_{1max}$). As an example, with the first gain being kept at the minimum value $G_{1min}$, the first exposure time $T_1$ may be adjusted by calculating $T_{1c}=T_1' \times G_1'/G_{1min}$ from a first exposure

amount ($T_1' \times G_1'$) of the previous frame and decrementing the first exposure time $T_1$ from $T_{1c}$ until the current brightness value $B_1$ of the first video stream shifts into the first target brightness range ($B_{1min}$, $B_{1max}$).

Likewise, a process of tuning the current brightness value $B_2$ into the predefined second target brightness range ($B_{2min}$, $B_{2max}$) through adjusting the second exposure time $T_2$ and the second gain $G_2$ may include the steps as follows.

On the one hand, when the current brightness value $B_2$ of the second video stream is below the lower limit $B_{2min}$ of the second target brightness range ($B_{2min}$, $B_{2max}$), the second exposure control means 422 increases the second exposure amount of the near-infrared image sensor 121 to adjust the current brightness value $B_2$ of the second video stream. First of all, the exposure time $T_2$ is increased, and it is determined whether a maximum value $T_{2max}$ of the second exposure time and a minimum value $G_{2min}$ of the second gain satisfy an exposure amount requirement of the second target brightness range ($B_{2min}$, $B_{2max}$).

If the requirement is satisfied, with the second gain being kept at the minimum value $G_{2min}$, the second exposure time $T_2$ is adjusted so that the current brightness value $B_2$ of the second video stream shifts into the second target brightness range ($B_{2mm}$, $B_{2max}$), and the minimum value $G_{2min}$ of the second gain and the adjusted second exposure time $T_2$ are output to the second drive unit 320.

If the requirement is not satisfied, the second gain $G_2$ is increased, and it is determined whether the maximum value $T_{2max}$ of the second exposure time and a maximum value $G_{2max}$ of the second gain satisfy the exposure amount requirement of the second target brightness range ($B_{2min}$, $B_{2max}$). If the requirement is still not satisfied, then the current values of the second exposure time $T_2$ and the second gain $G_2$ are maintained, and the second exposure control means 422 feeds the information indicating that the current brightness value $B_2$ remains outside of the second target brightness range ($B_{2min}$, $B_{2max}$) (e.g., the information may indicate that the second exposure time $T_2$ is at the maximum value $T_{2max}$ and the second gain $G_2$ at the maximum value $G_{2max}$) to the second illumination adjustment means 423. If the requirement is satisfied, then with the second exposure time being kept at the maximum value $T_{2max}$, the second gain $G_2$ is adjusted to tune the current brightness value $B_2$ of the second video stream into the second target brightness range ($B_{2mm}$, $B_{2max}$), and the maximum value $T_{2max}$ of the second exposure time and the adjusted second gain $G_2$ are output to the second drive unit 320.

As an example, with the second gain being kept at the minimum value $G_{2min}$, the second exposure time $T_2$ may be adjusted by calculating $T_{2c}=T_2' \times G_2'/G_{2\,min}$ from a second exposure amount ($T_2' \times G_2'$) of the previous frame and incrementing the second exposure time $T_2$ from $T_{2c}$ until the current brightness value $B_2$ of the second video stream shifts into the second target brightness range ($B_{2mm}$, $B_{2max}$). As an example, with the second exposure time being kept at the maximum value $T_{2max}$, the second gain $G_2$ may be adjusted by calculating $G_{2c}=T_2' \times G_2'/T_{2max}$ from a second exposure amount ($T_2' \times G_2'$) of the previous frame and incrementing the second gain $G_2$ from $G_{2c}$ until the current brightness value $B_2$ of the second video stream shifts into the second target brightness range ($B_{2mm}$, $B_{2max}$).

On the other hand, when the current brightness value $B_2$ of the second video stream is above the upper limit $B_{2max}$ of the second target brightness range ($B_{2mm}$, $B_{2max}$), the second exposure control means 422 reduces the second exposure amount of the near-infrared image sensor 121 to adjust the current brightness value $B_2$ of the second video stream. Specifically, first of all, the second gain $G_2$ is reduced, and it is determined whether the maximum value $T_{2max}$ of the second exposure time and the minimum value $G_{2min}$ of the second gain satisfy an exposure amount requirement of the second target brightness range ($B_{2min}$, $B_{2max}$). If the requirement is satisfied, with the second exposure time being kept at the maximum value $T_{2max}$, the second gain $G_2$ is adjusted so that the current brightness value $B_2$ of the second video stream shifts into the second target brightness range ($B_{2min}$, $B_{2max}$), and the maximum value $T_{2max}$ of the second exposure time and the adjusted second gain $G_2$ are output to the second drive unit 320.

If the requirement is not satisfied, the second exposure time $T_2$ is reduced, and it is determined whether a minimum value $T_{2min}$ of the second exposure time and the minimum value $G_{2min}$ of the second gain satisfy the exposure amount requirement of the second target brightness range ($B_{2min}$, $B_{2max}$). If the requirement is still not satisfied, then the current values of the second exposure time $T_2$ and the second gain $G_2$ are maintained, and the second exposure control means 422 feeds the information indicating that the current brightness value $B_2$ remains outside of the target brightness range ($B_{2mm}$, $B_{2max}$) (e.g., the information may indicate that the second exposure time $T_2$ is at the minimum value $T_{2min}$ and the second gain $G_2$ at the minimum value $G_{2min}$) to the second illumination adjustment means 423 that is connected to the illumination module 200. If the requirement is satisfied, then with the second gain being kept at the minimum value $G_{2min}$, the second exposure time $T_2$ is adjusted to tune the current brightness value $B_2$ of the second video stream into the second target brightness range ($B_{2min}$, $B_{2max}$), and the minimum value $G_{2min}$ of the second gain and the adjusted second exposure time $T_2$ are output to the second drive unit 320.

As an example, with the second exposure time being kept at the maximum value $T_{2max}$, the second gain $G_2$ may be adjusted by calculating $G_{2c}=T_2' \times G_2'/T_{2max}$ from a second exposure amount ($T_2' \times G_2'$) of the previous frame and decrementing the second gain $G_2$ from $G_{2c}$ until the current brightness value $B_2$ of the second video stream shifts into the second target brightness range ($B_{2min}$, $B_{2max}$). As an example, with the second gain being kept at the minimum value $G_{2min}$, the second exposure time $T_2$ may be adjusted by calculating $T_{2c}=T_2' \times G_2'/G_{2min}$ from a second exposure amount $(T_2' \times G_2')$ of the previous frame and decrementing the second exposure time $T_2$ from $T_{2c}$ until the current brightness value $B_2$ of the second video stream shifts into the second target brightness range $(B_{2mm}, B_{2max})$.

In other words, in this embodiment, the first exposure control means 412 tunes the current brightness value $B_1$ of the first video stream into the first target brightness range $(B_{1min}, B_{1max})$ through adjusting the first gain $G_1$ and the first exposure time $T_1$ and outputs the adjusted first gain $G_1$ and first exposure time $T_1$ to the first drive unit 310, which then drives, based on the received first gain $G_1$ and first exposure time $T_1$, the visible-light image sensor 111 to capture a visible-light scene of the tissue of interest 600 so that a brightness value of each subsequently captured image of the visible-light scene lies within the first target brightness range $(B_{1min}, B_{1max})$. Moreover, the second exposure control means 422 tunes the current brightness value $B_2$ of the second video stream into the second target brightness range $(B_{2min}, B_{2max})$ through adjusting the second gain $G_2$ and the second exposure time $T_2$ and outputs the adjusted second gain $G_2$ and second exposure time $T_2$ to the second drive unit 320, which then drives, based on the received second gain $G_2$ and second exposure time $T_2$, the near-infrared image sensor 121 to capture a near-infrared scene of the tissue of interest 600 so that a brightness value of each subsequently captured image of the near-infrared scene lies within the second target brightness range $(B_{2min}, B_{2max})$. In other embodiments of the present invention, the current brightness values of the first and second video streams may be adjusted otherwise. In an alternative embodiment, the endoscope may be a 3D endoscope. In this case, the first exposure control means 412 may include a first visible-light control unit and a second visible-light control unit, and the second exposure control means 422 may include a first near-infrared control unit and a second near-infrared control unit. Additionally, the first drive unit 310 may include a first visible-light drive unit 311 and a second visible-light drive unit 312, and the second drive unit 320 may include a first near-infrared drive unit 321 and a second near-infrared drive unit 322. The first visible-light drive unit 311 and the second visible-light drive unit 312 may be configured to drive the first visible-light image sensor 111A and the second visible-light image sensor 111B to capture the first visible-light scene images and the second visible-light scene images, respectively. Preferably, there is a horizontal parallax between the first and second visible-light scene images, which is compatible with the characteristics of the human eye, thereby providing a 3D visual effect. The first near-infrared drive unit 321 and the second near-infrared drive unit 322 may be configured to drive the first near-infrared image sensor 121A and the second near-infrared image sensor 121B to capture the first near-infrared scene images and the second near-infrared scene images, respectively. Preferably, there is a horizontal parallax between the first and second near-infrared scene images, which is compatible with the characteristics of the human eye, thereby providing a 3D visual effect. In this embodiment, in the bleeding mode, only either of the first and second visible-light control units may be activated to tune a current brightness value of the first or second visible-light video stream through adjusting the first exposure time $T_1$ and the first gain $G_1$. Alternatively, only either of the first and second visible-light control units may be activated to tune both the current brightness value $B_{11}$ of the first visible-light video stream and the current brightness value $B_{12}$ of the second visible-light video stream through adjusting the first exposure time $T_1$ and the first gain $G_1$. Still alternatively, both the first and second visible-light control units may be activated to tune the current brightness value $B_{11}$ of the first visible-light video stream and the current brightness value $B_{12}$ of the second visible-light video stream through adjusting separate pairs of first exposure time $T_1$ and first gain $G_1$. Likewise, only either of the first and second near-infrared control units may be activated to tune a current brightness value of the first or second near-infrared video stream through adjusting the second exposure time $T_2$ and the second gain $G_2$. Alternatively, only either of the first and second near-infrared control units may be activated to tune both the current brightness value $B_{21}$ of the first near-infrared video stream and the current brightness value $B_{22}$ of the second near-infrared video stream through adjusting the second exposure time $T_2$ and the second gain $G_2$. Still alternatively, both the first and second near-infrared control units may be activated to tune the current brightness value $B_{21}$ of the first near-infrared video stream and the current brightness value $B_{22}$ of the second near-infrared video stream through adjusting separate pairs of second exposure time $T_2$ and second gain $G_2$.

The scene fusion module 500 is configured to, when the current brightness value $B_1$ of the first video stream lies within the predefined first target brightness range $(B_{1min}, B_{1max})$ and/or the current brightness value $B_2$ of the second video stream lies within the predefined second target brightness range $(B_{2min}, B_{2max})$, be based on brightness information of the current image frame in the first video stream and brightness information of the current image frame in the second video stream to fuse the current image frames of the first and second video streams to obtain a brightness-fused image. Moreover, it is based on chroma information of the current image frame in the first video stream to fuse the brightness-fused image with the current image frame in the first video stream, thereby obtaining a scene-fused image. In the bleeding mode of the fluorescence endoscopy system, when the current brightness value $B_1$ of the first video stream lies within the first target brightness range $(B_{1min}, B_{1max})$ and/or the current brightness value $B_2$ of the second video stream lies within the second target brightness range $(B_{2min}, B_{2max})$, the scene fusion module 500 fuses the current image frames of the first and second video streams. In addition, the scene fusion module 500 is communicatively connected to the central controller 800. Upon receiving a command for activating the bleeding mode, the central controller 800 controls the scene fusion module 500 to fuse the current image frames of the first and second video streams, thereby obtain a scene-fused image. Further, upon receiving a command for activating the fluorescence mode, the central controller 800 controls the scene fusion module 500 to directly output the received first and second video streams. In this way, according to this embodiment, scene images captured by the sensors of different types can be fused to produce fused images of the tissue of interest 600 with effectively reduced blur due to camera movement. In this way, a user can be avoided from making an erroneous determination of a lesion due to image blur, resulting in increased surgical accuracy and safety. Moreover, an effectively improved signal-to-noise ratio can be obtained, which allows more details to be discerned from the images.

Specifically, the scene fusion module 500 includes an image fusion unit configured to obtain the brightness-fused image by fusing pixels of the current image frame in the first video stream with respective pixels of the current image frame in the second video stream based on the brightness information of the current image frame in the first video stream and the brightness information of the current image frame in the second video stream and to obtain the scene-fused image by fusing the brightness-fused image with the current image frame in the second video stream based on the chroma information of the current image frame in the second video stream.

Additionally, the scene fusion module 500 is communicatively connected to both the visible-light image sensor 111 and the near-infrared image sensor 121 in order to be able to acquire the first and second video streams. In case of the first video stream being output in a YUV or $YC_bC_r$ format, the image fusion unit is configured to take Y values of the pixels of the current image frame in the first video stream as their brightness values and take U, V, $C_b$ or $C_r$ values of the pixels of the current image frame in the first video stream as their chroma values. In case of the first video stream being output in a RAW or RGB format, the scene fusion module may further include an image mode switching unit configured to convert the current image frame in the first video stream into the YUV or $YC_bC_r$ space and then take Y values of the pixels of the current image frame in the first video stream as their brightness values and take U, V, $C_b$ or $C_r$ values of the pixels of the current image frame in the first video stream as their chroma values. The conversion may be accomplished in a similar manner as the above-described conversion of the video stream from the RGB format to the YUV or $YC_bC_r$ format during the acquisition of the current brightness value $B_1$ of the first video stream by the first brightness acquisition means 411 and, therefore, needs not to be described in further detail herein. The brightness information of the current image frame in the second video stream may be acquired in a similar manner as the above-described acquisition of the brightness information of the current image frame in the first video stream, or otherwise acquired taking advantage of the nature of near-infrared images, which is, however, not described in further detail herein for the sake of brevity.

The present invention is not limited to any particular algorithm for accomplishing the fusion based on the brightness values of the pixels of the current image frame in the first video stream and the brightness values of the respective pixels of the current image frame in the second video stream, and, for example, an arithmetic averaging algorithm, a weighted averaging algorithm, an extreme-value (maximum or minimum) finding algorithm, or the like. Preferably, weights for the brightness values of the pixels of the current image frame in the first video stream and weights for the brightness values of the respective pixels of the current image frame in the second video stream are derived from the brightness values of the pixels of the current image frame in the first video stream and the brightness values of the respective pixels of the current image frame in the second video stream according to a predefined normal weight distribution as a function of brightness. Moreover, the brightness values of the pixels of the current image frame in the first video stream and the brightness values of the respective pixels of the current image frame in the second video stream are weighted with the weights for the brightness values of the pixels of the current image frame in the first video stream and the weights for the brightness values of the respective pixels of the current image frame in the second video stream, respectively, deriving brightness values of pixels of the brightness-fused image.

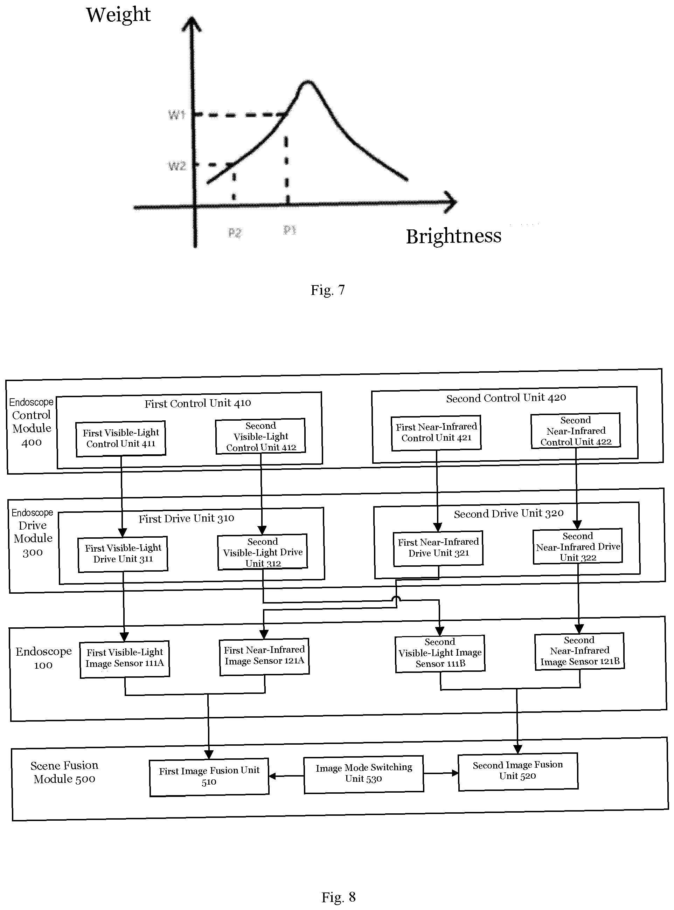

Fig. 7 schematically illustrates the normal weight distribution as a function of brightness according to an embodiment of the present invention, in which $P_1$ denotes a first brightness value; $P_2$, a second brightness value; $W_1$, a weight for the first brightness value $P_1$; and $W_2$, a weight for the second brightness value $P_2$. The normal distribution is a curve whose parameters can be set, preferably, with a mathematical expectation $\mu$=128 and a variance $\delta$=50. A brightness value $P_3$ of the brightness-fused image, a first brightness value $P_1$ of a respective corresponding pixel of the current image frame in the first video stream and a second brightness value $P_2$ of a respective corresponding pixel of the current image frame in the second video stream satisfy:

$$P_3 = W_1 P_1 + W_2 P_2$$

As shown in Fig. 7, a brightness value closer to the mathematical expectation is assigned with a larger weight. In this way, the brightness-fused image obtained by fusing the brightness information of the current image frame in the first video stream and the brightness information of the current image frame in the second video stream using the above normal distribution has suitable brightness values.

As shown in Fig. 1, when the endoscope is a 3D endoscope, the scene fusion module 500 is configured to obtain a first brightness-fused image through fusing a current image frame in the first visible-light video stream with a current image frame in the first near-infrared video stream based on brightness information of the current image frame in the first visible-light video stream and brightness information of the current image frame in the first near-infrared video stream and obtain a first scene-fused image by fusing the first brightness-fused image with the current image frame in the first visible-light video stream based on chroma information of the current image frame in the first visible-light video stream. It is also configured to obtain a second brightness-fused image through fusing a current image frame in the second visible-light video stream with a current image frame in the second near-infrared video stream based on brightness information of the current image frame in the second visible-light video stream and brightness information of the current image frame in the second near-infrared video stream and obtain a second scene-fused image by fusing the second brightness-fused image with the current image frame in the second visible-light video stream based on chroma information of the current image frame in the second visible-light video stream. Since the first visible-light and near-infrared scene images are captured from a single optical channel and the second visible-light and near-infrared scene images are captured from another single optical channel, and because of the horizontal parallax between the first and second visible-light scene images and between the first and second near-infrared scene images, there is also the horizontal parallax between the first and second scene-fused images that is compatible with the characteristics of the human eye, thereby providing a 3D visual effect.

Specifically, reference is now made to Fig. 8, a schematic illustration of image fusion in the bleeding mode according to an embodiment of the present invention. As shown in Fig. 8, the image fusion unit may include a first image fusion unit 510 and a second image fusion unit 520. The first image fusion unit 510 is configured to obtain the first brightness-fused image through fusing pixels of the current image frame in the first visible-light video stream with respective pixels of the current image frame in the first near-infrared video stream based on the brightness information of the current image frame in the first visible-light video stream and the brightness information of the current image frame in the first near-infrared video stream and obtain the first scene-fused image through fusing the first brightness-fused image with the current image frame in the first visible-light video stream based on the chroma information of the current image frame in the first visible-light video stream. The second image fusion unit 520 is configured to obtain the second brightness-fused image through fusing pixels of the current image frame in the second visible-light video stream with respective pixels of the current image frame in the second near-infrared video stream based on the brightness information of the current image frame in the second visible-light video stream and the brightness information of the current image frame in the second near-infrared video stream and obtain the second scene-fused image through fusing the second brightness-fused image with the current image frame in the second visible-light video stream based on the chroma information of the current image frame in the second visible-light video stream. The obtainment of the scene-fused images may be accomplished in a similar manner as described above and, therefore, needs not to be described in further detail herein.

Preferably, the fluorescence endoscopy system further includes a video pipeline 700 and the central controller 800. The central controller 800 includes a video overlay unit 810, a user input device 820 and a user interface 830. Scene-fused images output from the scene fusion module 500 are transmitted to the video pipeline 700, and the video overlay unit 810 produces a 3D image by overlaying the scene-fused images in the video pipeline 700. The user input device 820 is configured to receive operational commands from an operator, such as those for operating mode switching, and transfers them to the user interface 830. The user interface 830 is configured to control the scene fusion module 500, the endoscope control module 400, the illumination module 200 and the like with the received operational commands and control commands generated according to internal control conditions of the system. The 3D image produced by the video overlay unit 810 is transmitted by the user interface 830 to a monitor 900 in a surgeon's console and displayed thereon.

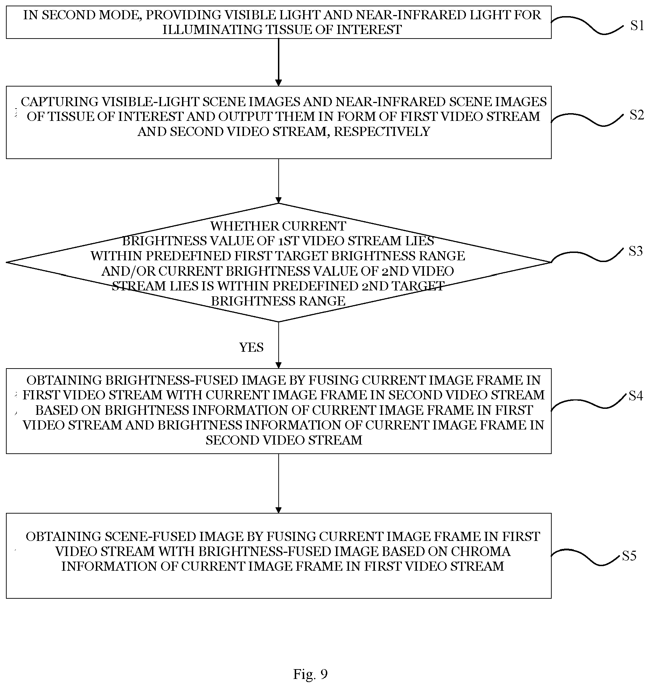

In correspondence with the fluorescence endoscopy system as defined above, the present invention also provides a control method of a fluorescence endoscopy system. Fig. 9 schematically illustrates a flowchart of a process for controlling the fluorescence endoscopy system in a bleeding mode according to an embodiment of the present invention. As shown in Fig. 9, operating modes of the fluorescence endoscopy system includes a first mode and a second mode. The process includes the steps of:

(S1) in the second mode, providing visible light and near-infrared light for illuminating tissue of interest;

(S2) capturing visible-light scene images and near-infrared scene images of the tissue of interest and outputting them in the form of a first video stream and a second video stream, respectively; and

(S3) determining whether a current brightness value $B_1$ of the first video stream lies within a predefined first target brightness range ($B_{1min}$, $B_{1max}$) and/or whether a current brightness value $B_2$ of the second video stream lies within a predefined second target

brightness range ($B_{2min}$, $B_{2max}$).

If the current brightness value $B_1$ of the first video stream lies within the predefined first target brightness range ($B_{1min}$, $B_{1max}$) and/or the current brightness value $B_2$ of the second video stream lies within the predefined second target brightness range ($B_{2mm}$, $B_{2max}$), the process further includes the steps of:

(S4) obtaining a brightness-fused image by fusing the current image frame in the first video stream with the current image frame in the second video stream based on brightness information of a current image frame in the first video stream and brightness information of a current image frame in the second video stream; and

(S5) obtaining a scene-fused image by fusing the current image frame in the first video stream with the brightness-fused image based on chroma information of the current image frame in the first video stream.

Thus, according to this embodiment, the fluorescence endoscopy system includes two modes, and in the second mode, when the current brightness value(s) of the first and/or second video stream(s) lie(s) within the respective target brightness range(s), the scene-fused image obtained by fusing the current visible-light and near-infrared image frames has a suitable brightness value, which results in an effectively increased signal-to-noise ratio, allows rich details to be discerned and can effectively reduce image blur arising from camera movement. In this way, a user can be avoided from making an erroneous determination of a lesion due to image blur, resulting in increased surgical accuracy and safety. Moreover, the fluorescence endoscopy system is based on conventional hardware and can have various functions by making slight modifications thereto, which can meet various surgical needs of physicians and enable easy surgical operation.

Preferably, in case of the endoscope being implemented as a 3D endoscope, capturing the visible-light scene images and near-infrared scene images of the tissue of interest and outputting them in the form of the first video stream and the second video stream, respectively, includes:
capturing first visible-light scene images, second visible-light scene images, first near-infrared scene images and second near-infrared scene images of the tissue of interest and outputting them in the form of a first visible-light video stream, a second visible-light video stream, a first near-infrared video stream and a second near-infrared video stream, respectively.

Moreover, determining whether the current brightness value $B_1$ of the first video stream lies within the predefined first target brightness range ($B_{1min}$, $B_{1max}$) includes:

determining whether current brightness value(s) of the first and/or second visible-light video stream(s) lie(s) within the predefined first target brightness range ($B_{1min}$, $B_{1max}$).

Additionally, determining whether the current brightness value $B_2$ of the second video stream lies within the predefined second target brightness range ($B_{2min}$, $B_{2max}$) includes:
determining whether current brightness value(s) of the first and/or second near-infrared video stream(s) lie(s) within the predefined second target brightness range ($B_{2mm}$, $B_{2max}$).

Further, obtaining the brightness-fused image by fusing the current image frame in the first video stream with the current image frame in the second video stream based on the brightness information of the current image frame in the first video stream and the brightness information of the current image frame in the second video stream includes:
obtaining a first brightness-fused image by fusing a current image frame in the first visible-light video stream with a current image frame in the first near-infrared video stream based on brightness information of the current image frame in the first visible-light video stream and brightness information of the current image frame in the first near-infrared video stream and obtaining a second brightness-fused image by fusing a current image frame in the second visible-light video stream with a current image frame in the second near-infrared video stream based on brightness information of the current image frame in the second visible-light video stream and brightness information of the current image frame in the second near-infrared video stream.

Furthermore, obtaining the scene-fused image by fusing the current image frame in the first video stream with the brightness-fused image based on the chroma information of the current image frame in the first video stream includes:
obtaining a first scene-fused image by fusing the current image frame in the first visible-light video stream and the first brightness-fused image based on chroma information of the current image frame in the first visible-light video stream and obtaining a second scene-fused image by fusing the current image frame in the second visible-light video stream with the second brightness-fused image based on chroma information of the current image frame in the second visible-light video stream.

Preferably, if the current brightness value $B_1$ of the first video stream is not in the first target brightness range ($B_{1min}$, $B_{1max}$), then a first exposure amount is adjusted so that the current brightness value $B_1$ of the first video stream shifts into the first target brightness range ($B_{1min}$, $B_{1max}$), wherein the first exposure amount is the product of a first exposure time $T_1$ and a first gain $G_1$.

Moreover, if the current brightness value $B_2$ of the second video stream is not in the second target brightness range ($B_{2min}$, $B_{2max}$), then a second exposure amount is adjusted so that the current brightness value

$B_2$ of the second video stream shifts into the second target brightness range $(B_{2min}, B_{2max})$, wherein the second exposure amount is the product of a second exposure time $T_2$ and a second gain $G_2$.

Preferably, when the current brightness value $B_1$ of the first video stream is below the lower limit $B_{1min}$ of the first target brightness range $(B_{1min}, B_{1max})$, the first exposure amount of the visible-light image sensor is increased to adjust the current brightness value $B_1$ of the first video stream.

Preferably, adjusting the current brightness value $B_1$ of the first video stream by increasing the first exposure amount of the visible-light image sensor includes: determining whether a maximum value $T_{1max}$ of the first exposure time and a minimum value $G_{1min}$ of the first gain satisfy an exposure amount requirement of the first target brightness range $(B_{1min}, B_{1max})$.

If the requirement is satisfied, with the first gain being kept at the minimum value $G_{1min}$, the first exposure time $T_1$ is adjusted to tune the current brightness value $B_1$ of the first video stream into the first target brightness range $(B_{1min}, B_{1max})$.

If the requirement is not satisfied, the first gain $G_1$ is increased, and it is determined whether the maximum value $T_{1max}$ of the first exposure time and a maximum value $G_{1max}$ of the first gain satisfy the exposure amount requirement of the first target brightness range $(B_{1min}, B_{1max})$.

If the requirement is satisfied, with the first exposure time being kept at the maximum value $T_{1max}$, the first gain $G_1$ is adjusted to tune the current brightness value $B_1$ of the first video stream into the first target brightness range.

Preferably, when the current brightness value $B_1$ of the first video stream is above the upper limit $B_{1max}$ of the first target brightness range $(B_{1min}, B_{1max})$, the first exposure amount of the visible-light image sensor 111 is reduced to adjust the current brightness value $B_1$ of the first video stream.

Preferably, adjusting the current brightness value $B_1$ of the first video stream by reducing the first exposure amount of the visible-light image sensor 111 includes: determining whether the maximum value $T_{1max}$ of the first exposure time and the minimum value $G_{1min}$ of the first gain satisfy an exposure amount requirement of the first target brightness range $(B_{1min}, B_{1max})$.

If the requirement is satisfied, with the first exposure time being kept at the maximum value $T_{1max}$, the first gain $G_1$ is adjusted to tune the current brightness value $B_1$ of the first video stream into the first target brightness range $(B_{1min}, B_{1max})$.

If the requirement is not satisfied, the first exposure time $T_1$ is reduced, and it is determined whether a minimum value $T_{1min}$ of the first exposure time and the minimum value $G_{1min}$ of the first gain satisfy the exposure amount requirement of the first target brightness range $(B_{1min}, B_{1max})$.

If the requirement is satisfied, with the first gain being kept at the minimum value $G_{1min}$, the first exposure time $T_1$ is adjusted to tune the current brightness value $B_1$ of the first video stream into the first target brightness range.

Preferably, when the current brightness value $B_2$ of the second video stream is below the lower limit of the second target brightness range $(B_{2min}, B_{2max})$, the second exposure amount of the near-infrared image sensor 121 is increased to adjust the current brightness value $B_2$ of the second video stream.

Preferably, adjusting the current brightness value $B_2$ of the second video stream by increasing the second exposure amount of the near-infrared image sensor 121 includes: determining whether a maximum value $T_{2max}$ of the second exposure time and a minimum value $G_{2min}$ of the second gain satisfy an exposure amount requirement of the second target brightness range $(B_{2min}, B_{2max})$.

If the requirement is satisfied, with the second gain being kept at the minimum value $G_{2min}$, the second exposure time $T_2$ is adjusted to tune the current brightness value $B_2$ of the second video stream into the second target brightness range $(B_{2mm}, B_{2max})$.

If the requirement is not satisfied, the second gain $G_2$ is increased, and it is determined whether the maximum value $T_{2max}$ of the second exposure time and a maximum value $G_{2max}$ of the second gain satisfy the exposure amount requirement of the second target brightness range $(B_{2min}, B_{2max})$.

If the requirement is satisfied, with the second exposure time being kept at the maximum value $T_{2max}$, the second gain $G_2$ is adjusted to tune the current brightness value $B_2$ of the second video stream into the second target brightness range $(B_{2mm}, B_{2max})$.

Preferably, when the current brightness value $B_2$ of the second video stream is above the upper limit $B_{2max}$ of the second target brightness range $(B_{2min}, B_{2max})$, the second exposure amount of the near-infrared image sensor 121 is reduced to adjust the current brightness value $B_2$ of the second video stream.

Preferably, adjusting the current brightness value $B_2$ of the second video stream by reducing the second exposure amount of the near-infrared image sensor 121 includes: determining whether the maximum value $T_{2max}$ of the second exposure time and the minimum value $G_{2min}$ of the second gain satisfy an exposure amount requirement of the second target brightness range $(B_{2min}, B_{2max})$.

If the requirement is satisfied, with the second exposure time being kept at the maximum value $T_{2max}$, the second gain $G_2$ is adjusted to tune the current brightness value $B_2$ of the second video stream into the second target brightness range $(B_{2min}, B_{2max})$.

If the requirement is not satisfied, the second exposure time $T_2$ is reduced, and it is determined whether a minimum value $T_{2min}$ of the second exposure time and the minimum value $G_{2min}$ of the second gain satisfy the exposure amount requirement of the second target

brightness range ($B_{2min}$, $B_{2max}$).

If the requirement is satisfied, with the second gain being kept at the minimum value $G_{2min}$, the second exposure time $T_2$ is adjusted to tune the current brightness value $B_2$ of the second video stream into the second target brightness range ($B_{2mm}$, $B_{2max}$).

Preferably, if the current brightness value $B_1$ of the first video stream cannot be tuned into the first target brightness range ($B_{1min}$, $B_{1max}$) by adjusting the first gain $G_1$ and the first exposure time $T_1$, output power of a first light source module which provides the visible light is adjusted to shift the current brightness value $B_1$ of the first video stream into the first target brightness range ($B_{1min}$, $B_{1max}$).

Preferably, if the current brightness value $B_2$ of the second video stream cannot be tuned into the second target brightness range ($B_{2min}$, $B_{2max}$) by adjusting the second gain $G_2$ and the second exposure time $T_2$, output power of a third light source module which provides the near-infrared light is adjusted to shift the current brightness value $B_2$ of the second video stream into the second target brightness range ($B_{2min}$, $B_{2max}$).

Preferably, obtaining the brightness-fused image by fusing the current image frame in the first video stream with the current image frame in the second video stream based on the brightness information of the current image frame in the first video stream and the brightness information of the current image frame in the second video stream includes: obtaining the brightness-fused image by fusing pixels of the current image frame in the first video stream with respective pixels of the current image frame in the second video stream based on the brightness information of the current image frame in the first video stream and the brightness information of the current image frame in the second video stream.

Preferably, obtaining the scene-fused image by fusing the current image frame in the first video stream with the brightness-fused image based on the chroma information of the current image frame in the first video stream includes: assigning chroma information of the pixels of the current image frame in the first video stream to the respective pixels of the brightness-fused image as their chroma values.

Preferably, in case of the first video stream being output in a RAW or RGB format, before the fusion of the current image frame in the first video stream with the current image frame in the second video stream, the method further includes: converting the current image frame in the first video stream into the YUV or $YC_bC_r$ space.

Preferably, fusing the pixels of the current image frame in the first video stream with the respective pixels of the current image frame in the second video stream based on the brightness information of the current image frame in the first video stream and the brightness information of the current image frame in the second video stream includes:

deriving weights for brightness values of the pixels of the current image frame in the first video stream and weights for brightness values of the respective pixels of the current image frame in the second video stream from the brightness values of the pixels of the current image frame in the first video stream and the brightness values of the respective pixels of the current image frame in the second video stream according to a predefined normal weight distribution as a function of brightness, respectively; and

weighting the brightness values of the pixels of the current image frame in the first video stream and the brightness values of the respective pixels of the current image frame in the second video stream with the derived weights for the brightness values of the pixels of the current image frame in the first video stream and weights for the brightness values of the respective pixels of the current image frame in the second video stream, respectively, thereby obtaining brightness values of pixels of the brightness-fused image.

Based on the same inventive concept, the present invention also provides a storage medium storing thereon a computer program, which, when executed by a processor, implements the control method as defined above.

Thus, according to this embodiment, the fluorescence endoscopy system includes two modes, and in the second mode, when the current brightness value(s) of the first and/or second video stream(s) lie(s) within the respective target brightness range(s), the scene-fused image obtained by fusing the current visible-light and near-infrared image frames has a suitable brightness value, which results in an effectively increased signal-to-noise ratio, allows rich details to be discerned and can effectively reduce image blur arising from camera movement. In this way, a user can be avoided from making an erroneous determination of a lesion due to image blur, resulting in increased surgical accuracy and safety. Moreover, the fluorescence endoscopy system is based on conventional hardware and can have various functions by making slight modifications thereto, which can meet various surgical needs of physicians and enable easy surgical operation.

According to embodiments of the present invention, the storage medium may be implemented as any combination of one or more computer-readable media each in the form of a computer-readable signal medium or a computer-readable storage medium. The computer-readable storage medium may be, for example, but is not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. As used herein, the phase "computer-readable storage medium" may refer to any tangible medium that can contain, or store a program for use by or in connection with an instruction execution system, apparatus, or device.

The computer-readable signal medium may include a propagated data signal with computer-readable program code embodied therein, for example, in baseband or as part of a carrier wave. Such a propagated signal may take any of a variety of forms, including, but not limited to, electro-magnetic, optical, or any suitable combination thereof. The computer-readable signal medium may also be any computer-readable medium that is not a computer-readable storage medium and that can communicate, propagate, or transport a program for use by or in connection with an instruction execution system, apparatus, or device.

Computer program code for carrying out operations of the present invention may be written in any combination of one or more programming languages, including an object-oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on a remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

In summary, compared with the prior art, the fluorescence endoscopy system, the control method and the storage medium proposed in the present invention have the advantages as follows: in the first mode, in addition to visible light, the illumination module can also provide excitation light, which, when irradiated on tissue of interest, can excite the tissue of interest to produce fluorescence, thereby helping an operator to obtain by observation information of the tissue that cannot be obtained under visible light illumination. In the second mode, the illumination module can provide both visible light and near-infrared light for illuminating tissue of interest, and the scene fusion module is configured to, when the current brightness value(s) of the first and/or second video stream(s) lie(s) within the respective target brightness range(s), the scene-fused image obtained by fusing the current visible-light and near-infrared image frames has a suitable brightness value, which allows rich details to be discerned. Therefore, on the one hand, the fluorescence endoscopy system of the present invention can effectively reduce image blur arising from camera movement, avoiding a user from making an erroneous determination of a lesion due to image blur and hence resulting in increased surgical accuracy and safety. In addition, an effectively increased signal-to-noise ratio can be achieved to allow more details to be discerned and obtained from the image. On the other hand, the fluorescence endoscopy system of the present invention is based on conventional hardware and can have various functions by making slight modifications thereto, which can meet various surgical needs of physicians and enable easy surgical operation.

It is to be noted that the devices and methods disclosed in the embodiments therein may be implemented in other ways and the device embodiments described above are merely illustrative. For example, the flowcharts and block diagrams in the figures illustrate the architecture, functionality and operation of possible implementations of devices, methods, and computer program products according to various embodiments of the present invention. In this regard, each block in the flowcharts or block diagrams may represent a module, segment, or portion of codes, which comprises one or more executable instructions for implementing the specified logical function(s). It is to be also noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It is to be further noted that each block of the block diagrams and/or flowchart illustrations, and combinations of blocks in the block diagrams and/or flowchart illustrations, can be implemented by special purpose hardware-based systems that perform the specified functions or acts or carry out combinations of special purpose hardware and computer instructions.

Further, the various functional modules in the embodiments herein may be integrated into a discrete component, or provided as separate modules. Alternatively, two or more of the modules may be integrated into a discrete component.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A fluorescence endoscopy system, comprising an endoscope, an illumination module, an endoscope drive module and a scene fusion module,

   the fluorescence endoscopy system having operating modes including a first mode and a second mode,
   the endoscope comprising a visible-light image sensor and a near-infrared image sensor,
   wherein, in the first mode, the illumination module is configured to provide visible light for illuminating the tissue of interest and excitation light for exciting the tissue of interest to produce fluorescence, the visible-light image sensor is configured to capture visible-light scene images of the tissue of interest and output the visible-light scene images in a form of a first video stream, and the near-infrared image sensor is configured to capture fluorescence scene images of the tissue of interest and output the fluorescence scene images in a form of a second video stream,
   wherein, in the second mode, the illumination module is configured to provide visible light and near-infrared light both for illuminating the tissue of interest, the visible-light image sensor is configured to capture visible-light scene images of the tissue of interest and output the visible-light scene images in a form of a first video stream, and the near-infrared image sensor is configured to capture near-infrared scene images of the tissue of interest and output the near-infrared scene images in a form of a second video stream,
   wherein the endoscope drive module comprises a first drive unit and a second drive unit, wherein the first drive unit is configured to, in the second mode, drive the visible-light image sensor to capture the visible-light scene image according to a first exposure time and a first gain, and the second drive unit is configured to, in the second mode, drive the near-infrared image sensor to capture the near-infrared scene image according to a second exposure time and a second gain,
   wherein the scene fusion module is configured to, when a current brightness value of the first video stream lies within a predefined first target brightness range and/or a current brightness value of the second video stream lies within a predefined second target brightness range, obtain a brightness-fused image by fusing a current image frame in the first video stream with a current image frame in the second video stream based on brightness information of the current image frame in the first video stream and brightness information of the current image frame in the second video stream and obtain a scene-fused image by fusing the brightness-fused image with the current image frame in the first video stream based on chroma information of the current image frame in the first video stream.

2. The fluorescence endoscopy system according to claim 1, further comprising an endoscope control module, the endoscope control module comprising a first control unit and/or a second control unit, the first control unit configured to, in the second mode, cause the current brightness value of the first video stream to lie within the predefined first target brightness range, the second control unit configured to, in the second mode, cause the current brightness value of the second video stream to lie within the predefined second target brightness range.

3. The fluorescence endoscopy system according to claim 2,

   wherein the first control unit comprises:

      a first brightness acquisition means configured to acquire the current brightness value of the first video stream; and
      a first exposure control means configured to determine whether the current brightness value of the first video stream lies within the first target brightness range and, if the current brightness value of the first video stream does not lie within the first target brightness range, adjust a first exposure amount of the visible-light image sensor to cause the current brightness value of the first video stream to lie within the first target brightness range, and

   wherein the second control unit comprises:

      a second brightness acquisition means configured to acquire the current brightness value of the second video stream; and
      a second exposure control means configured to determine whether the current brightness value of the second video stream is within the second target brightness range and, if the current brightness value of the second video stream does not lie within the second target brightness range, adjust a second exposure amount of the near-infrared image sensor to cause the current brightness value of the second video stream to lie within the second target brightness range.

4. The fluorescence endoscopy system according to claim 3, wherein when the current brightness value of the first video stream is below a lower limit of the first target brightness range, the first exposure control means adjusts the current brightness value of the first video stream by increasing the first exposure amount of the visible-light image sensor, and

when the current brightness value of the second video stream is below a lower limit of the second target brightness range, the second exposure control means adjusts the current brightness value of the second video stream by increasing the second exposure amount of the near-infrared image sensor.

5. The fluorescence endoscopy system according to claim 4,

wherein the first exposure control means is configured to: determine whether a maximum value of the first exposure time and a minimum value of the first gain satisfy an exposure amount requirement of the first target brightness range;

if the requirement is satisfied, with the first gain being kept at the minimum value thereof, adjust the first exposure time to cause the current brightness value of the first video stream to lie within the first target brightness range; and

if the requirement is not satisfied, determine whether the maximum value of the first exposure time and a maximum value of the first gain satisfy the exposure amount requirement of the first target brightness range and, if so, with the first exposure time being kept at the maximum value thereof, adjust the first gain to cause the current brightness value of the first video stream to lie within the first target brightness range, and

wherein the second exposure control means is configured to: determine whether a maximum value of the second exposure time and a minimum value of the second gain satisfy an exposure amount requirement of the second target brightness range;

if the requirement is satisfied, with the second gain being kept at the minimum value thereof, adjust the second exposure time to cause the brightness value of the second video stream to lie within the second target brightness range; and

if the requirement is not satisfied, determine whether the maximum value of the second exposure time and a maximum value of the second gain satisfy the exposure amount requirement of the second target brightness range and, if so, with the second exposure time being kept at the maximum value thereof, adjust the second gain to cause the brightness value of the second video stream to lie within the second target brightness range.

6. The fluorescence endoscopy system according to claim 3, wherein when the current brightness value of the first video stream is above an upper limit of the first target brightness range, the first exposure control means adjusts the current brightness value of the first video stream by reducing the first exposure amount of the visible-light image sensor, and

when the current brightness value of the second video stream is above an upper limit of the second target brightness range, the second exposure control means adjusts the current brightness value of the second video stream by reducing the second exposure amount of the near-infrared image sensor.

7. The fluorescence endoscopy system according to claim 6,

wherein the first exposure control means is configured to: determine whether a maximum value of the first exposure time and a minimum value of the first gain satisfy an exposure amount requirement of the first target brightness range;

if the requirement is satisfied, with the first exposure time being kept at the maximum value thereof, adjust the first gain to cause the current brightness value of the first video stream to lie within the first target brightness range; and

if the requirement is not satisfied, determine whether a minimum value of the first exposure time and the minimum value of the first gain satisfy the exposure amount requirement of the first target brightness range and, if so, with the first gain being kept at the minimum value thereof, adjust the first exposure time to cause the current brightness value of the first video stream to lie within the first target brightness range, and

wherein the second exposure control means is configured to: determine whether a maximum value of the second exposure time and a minimum value of the second gain satisfy an exposure amount requirement of the second target brightness range;

if the requirement is satisfied, with the second exposure time being kept at the maximum value thereof, adjust the second gain to cause the brightness value of the second video stream to lie within the second target brightness range; and

if the requirement is not satisfied, determine whether a minimum value of the second exposure time and the minimum value of the second gain satisfy the exposure amount requirement of the second target brightness range and, if so, with the second gain being kept at the minimum value thereof, adjust the second exposure time to cause the brightness value of the second video stream to lie within the second target brightness range.

8. The fluorescence endoscopy system according to claim 3, wherein the illumination module comprises a first light source module configured to provide the visible light and a third light source module configured to provide the near-infrared light, wherein the first control unit further comprises a first illumination adjustment means configured to control, when it is impossible to cause the current brightness value of the first video stream to lie within the first target brightness range by adjusting the first gain and the first exposure time, the illumination module to adjust an output power of the first light source module to cause the current brightness value of the first video stream to lie within the first target brightness range, and wherein

the second control unit further comprises a second illumination adjustment means configured to control, when it is impossible to cause the current brightness value of the second video stream to lie within the second target brightness range by adjusting the second gain and the second exposure time, the illumination module to adjust output power of the third light source module to cause the current brightness value of the second video stream to lie within the second target brightness range.

9. The fluorescence endoscopy system according to claim 3, wherein

the first video stream is YUV or $YC_bC_r$- coded, the first brightness acquisition means is configured to take an average or weighted value of Y values of all or some pixels of the current image frame of the first video stream as the current brightness value, or
the first video stream is RAW- or RGB-coded, the first brightness acquisition means is configured to derive brightness values of the pixels of the current image frame of the first video stream from RGB values thereof and then take an average or weighted value of the brightness values of all or some pixels of the current image frame of the first video stream as the current brightness value.

10. The fluorescence endoscopy system according to claim 1, wherein the endoscope further comprises a beam-splitting prism group configured to separate the visible light reflected from the illuminated tissue of interest from the near-infrared light reflected from the illuminated tissue of interest in the second mode and separate the visible light reflected from the illuminated tissue of interest from the fluorescence produced by the excited tissue of interest in the first mode so that the reflected visible light can be captured by a photosensitive surface of the visible-light image sensor and that the reflected near-infrared light or the fluorescence can be captured by a photosensitive surface of the near-infrared image sensor.

11. The fluorescence endoscopy system according to claim 10, wherein the beam-splitting prism group comprises a first beam-splitting prism, a second beam-splitting prism, a visible-light band-pass filter and a near-infrared band-pass filter, the visible-light band-pass filter configured to allow passage of visible light therethrough and block light at other wavelengths, the near-infrared band-pass filter configured to allow passage of near-infrared light therethrough and block light at other wavelengths, the near-infrared band-pass filter disposed between the first beam-splitting prism and the second beam-splitting prism, a semi-transmissive, semi-reflective film is provided on a surface of the first beam-splitting prism adjacent to the second beam-splitting prism and configured to reflect part of light incident thereon and allow transmission of the rest of the incident light, wherein: the photosensitive surface of the visible-light image sensor is adjacent to an exit surface of the first beam-splitting prism; the visible-light band-pass filter is disposed between the exit surface of the first beam-splitting prism and the photosensitive surface of the visible-light image sensor; and the photosensitive surface of the near-infrared image sensor is adjacent to an exit surface of the second beam-splitting prism.

12. The fluorescence endoscopy system according to claim 1, wherein the scene fusion module comprises: an image fusion unit configured to obtain the brightness-fused image by fusing pixels of the current image frame in the first video stream with respective pixels of the current image frame in the second video stream based on the brightness information of the current image frame in the first video stream and the brightness information of the current image frame in the second video stream and obtain the scene-fused image by fusing the brightness-fused image with the current image frame in the first video stream based on the chroma information of the current image frame in the first video stream.

13. The fluorescence endoscopy system according to claim 12, wherein the image fusion unit is configured to: derive weights for brightness values of the pixels of the current image frame in the first video stream and weights for brightness values of the respective pixels of the current image frame in the second video stream from the brightness values of the pixels of the current image frame in the first video stream and the brightness values of the respective pixels of the current image frame in the second video stream, respectively, according to a predefined normal weight distribution as a function of brightness; and obtain brightness value of pixels of the brightness-fused image by weighting the brightness values of the pixels of the current image frame in the first video stream and the brightness values of the respective pixels of the current image frame in the second video stream with the derived weights for the brightness values of the pixels of the current image frame in the first video stream and weights for the brightness values of the respective pixels of the current image frame in the second video stream, respectively.

14. The fluorescence endoscopy system according to claim 11, wherein the scene fusion module further comprises an image mode switching unit configured to convert, in case of the first video stream being output in a RAW or RGB format, pixels of the current image frame in the first video stream into a YUV or $YC_bC_r$ space, take Y values of the pixels of the current image frame in the first video stream as brightness values of the pixels, and take U or V values or $C_b$ or $C_r$ values of the pixels of the current image frame in the first video stream as chroma values of the pixels of the current image frame in the first video stream.

15. The fluorescence endoscopy system according to claim 1, wherein the endoscope is a three-dimensional endoscope, wherein:

the visible-light image sensor comprises a first visible-light image sensor and a second visible-light image sensor;
the near-infrared image sensor comprises a first near-infrared image sensor and a second near-infrared image sensor;
the first video stream comprises a first visible-light video stream and a second visible-light video stream;
the second video stream comprises a first near-infrared video stream and a second near-infrared video stream;
in the second mode, the first visible-light image sensor is configured to capture first visible-light scene images of the tissue of interest and output the first visible-light scene images in a form of the first visible-light video stream, the second visible-light image sensor is configured to capture second visible-light scene images of the tissue of interest and output the second visible-light scene images in a form of the second visible-light video stream, the first near-infrared image sensor is configured to capture first near-infrared scene images of the tissue of interest and output first near-infrared scene images in a form of the first near-infrared video stream, and the second near-infrared image sensor is configured to capture second near-infrared scene images of the tissue of interest and output the second near-infrared scene images in a form of the second near-infrared video stream; and
the scene fusion module is configured to obtain:
a first brightness-fused image by fusing a current image frame in the first visible-light video stream with a current image frame in the first near-infrared video stream based on brightness information of the current image frame in the first visible-light video stream and brightness information of the current image frame in the first near-infrared video stream; a first scene-fused image by fusing the first brightness-fused image with the current image frame in the first visible-light video stream based on chroma information of the current image frame in the first visible-light video stream; a second brightness-fused image by fusing a current image frame in the second visible-light video stream with a current image frame in the second near-infrared video stream based on brightness information of the current image frame in the second visible-light video stream and the brightness information of the current image frame in the second near-infrared video stream; and a second scene-fused image by fusing the second brightness-fused image with the current image frame in the second visible-light video stream based on chroma information of the current image frame in the second visible-light video stream.

16. The fluorescence endoscopy system according to claim 2, further comprising a central controller, which, upon receiving a command for activating the first mode, controls the first control unit and the second control unit to adjust, according to the first target brightness range and the second target brightness range that are pre-configured in the endoscope control module, the current brightness values of the first video stream and the second video stream, or transmits the first target brightness range and the second target brightness range to the first control unit and the second control unit, respectively, so that the first control unit and the second control unit adjust, according to the first target brightness range and the second target brightness range that are pre-configured in the endoscope control module, the current brightness values of the first video stream and the second video stream.

17. The fluorescence endoscopy system according to claim 15, further comprising a central controller, the central controller comprising a video overlay unit configured to produce a three-dimensional image by overlaying scene-fused images output from the scene fusion module and transmitting the produced three-dimensional image to a monitor for display.

18. The fluorescence endoscopy system according to claim 1, wherein the first drive unit is further configured to drive the visible-light image sensor to capture the visible-light scene images according to a third exposure time and a third gain in the first mode, and the second drive unit is further configured to drive the near-infrared image sensor to capture the fluorescence scene images according to a fourth exposure time and a fourth gain in the first mode.

19. The fluorescence endoscopy system according to claim 2, wherein the first control unit is further configured to cause the current brightness value of the first video stream to lie within a predefined third target brightness range in the first mode, and/or the second control unit is further configured to cause the current brightness value of the second video stream to lie within a predefined fourth target brightness range in the first mode.

20. A control method for a fluorescence endoscopy system, wherein the fluorescence endoscopy system has operating modes including a first mode and a second mode, and the control method comprises:

in the second mode, providing visible light and near-infrared light for illuminating tissue of interest;

capturing visible-light scene images and near-infrared scene images of the tissue of interest and outputting the visible-light scene images in a form of a first video stream and the near-infrared scene images in a form of a second video stream;

determining whether a current brightness value of the first video stream lies within a predefined first target brightness range and/or whether a current brightness value of the second video stream lies within a predefined second target brightness range;

if the current brightness value of the first video stream lies within the predefined first target brightness range and/or the current brightness value of the second video stream lies within the predefined second target brightness range, then obtaining a brightness-fused image by fusing a current image frame in the first video stream with a current image frame in the second video stream based on brightness information of the current image frame in the first video stream and brightness information of the current image frame in the second video stream; and

obtaining a scene-fused image by fusing the current image frame in the first video stream with the brightness-fused image based on chroma information of the current image frame in the first video stream.

21. The control method for a fluorescence endoscopy system according to claim 20, wherein if the current brightness value of the first video stream does not lie in the first target brightness range, a first exposure amount is adjusted to cause the current brightness value of the first video stream to lie within the first target brightness range, wherein the first exposure amount is a product of a first exposure time and a first gain, and

if the current brightness value of the second video stream does not lie in the second target brightness range, a second exposure amount is adjusted to cause the current brightness value of the second video stream to lie within the second target brightness range, wherein the second exposure amount is a product of a second exposure time and a second gain.

22. The control method for a fluorescence endoscopy system according to claim 21, wherein when the current brightness value of the first video stream is below a lower limit of the first target brightness range, the current brightness value of the first video stream is adjusted by increasing the first exposure amount, and

when the current brightness value of the second video stream is below a lower limit of the second target brightness range, the current brightness value of the second video stream is adjusted by increasing the second exposure amount.

23. The control method for a fluorescence endoscopy system according to claim 22, wherein adjusting the current brightness value of the first video stream by increasing the first exposure amount comprises:

determining whether a maximum value of the first exposure time and a minimum value of the first gain satisfy an exposure amount requirement of the first target brightness range;

if the requirement is satisfied, with the first gain being kept at the minimum value thereof, adjusting the first exposure time to cause the current brightness value of the first video stream to lie within the first target brightness range; and

if the requirement is not satisfied, determining whether the maximum value of the first exposure time and a maximum value of the first gain satisfy the exposure amount requirement of the first target brightness range and, if so, with the first exposure time being kept at the maximum value thereof, adjusting the first gain to cause the current brightness value of the first video stream to lie within the first target brightness range, and wherein adjusting the current brightness value of the second video stream by increasing the second exposure amount comprises:

determining whether a maximum value of the second exposure time and a minimum value of the second gain satisfy an exposure amount requirement of the second target brightness range;

if the requirement is satisfied, with the second gain being kept at the minimum value thereof, adjusting the second exposure time to cause the brightness value of the second video stream to lie within the second target brightness range; and

if the requirement is not satisfied, determining whether the maximum value of the second exposure time and a maximum value of the second gain satisfy the exposure amount requirement of the second target brightness range and, if so, with the second exposure time being kept at the maximum value thereof, adjusting the second gain to cause the brightness value of the second video stream to lie within the second target brightness range.

24. The control method for a fluorescence endoscopy system according to claim 21, wherein when the current brightness value of the first video stream is above an upper limit of the first target brightness range, the current brightness value of the first video stream is adjusted by reducing the first exposure amount, and

when the current brightness value of the second video stream is above an upper limit of the second target brightness range, the current brightness value of the second video stream is adjusted by reducing the second exposure amount.

25. The control method for a fluorescence endoscopy system according to claim 24, wherein adjusting the current brightness value of the first video stream by reducing the first exposure amount comprises:

determining whether a maximum value of the first exposure time and a minimum value of the first gain satisfy an exposure amount requirement of the first target brightness range;

if the requirement is satisfied, with the first exposure time being kept at the maximum value thereof, adjusting the first gain to cause the current brightness value of the first video stream to lie within the first target brightness range; and

if the requirement is not satisfied, determining whether a minimum value of the first exposure time and the minimum value of the first gain satisfy the exposure amount requirement of the first target brightness range and, if so, with the first gain being kept at the minimum value thereof, adjusting the first exposure time to cause the current brightness value of the first video stream to lie within the first target brightness range, and

wherein adjusting the current brightness value of the second video stream by reducing the second exposure amount comprises:

determining whether a maximum value of the second exposure time and a minimum value of the second gain satisfy an exposure amount requirement of the second target brightness range;

if the requirement is satisfied, with the second exposure time being kept at the maximum value thereof, adjusting the second gain to cause the brightness value of the second video stream to lie within the second target brightness range; and

if the requirement is not satisfied, determining whether a minimum value of the second exposure time and the minimum value of the second gain satisfy the exposure amount requirement of the second target brightness range and, if so, with the second gain being kept at the minimum value thereof, adjusting the second exposure time to cause the brightness value of the second video stream to lie within the second target brightness range.

26. The control method for a fluorescence endoscopy system according to claim 21, wherein when it is impossible to cause the current brightness value of the first video stream to lie within the first target brightness range by adjusting the first gain and the first exposure time, an output power of a first light source module that provides the visible light is adjusted to cause the current brightness value of the first video stream to lie within the first target brightness range, and

when it is impossible to cause the current brightness value of the second video stream to lie within the second target brightness range by adjusting the second gain and the second exposure time, an output power of a third light source module that provides the near-infrared light is adjusted to cause the current brightness value of the second video stream to lie within the second target brightness range.

27. The control method for a fluorescence endoscopy system according to claim 20, wherein obtaining the brightness-fused image by fusing the current image frame in the first video stream with the current image frame in the second video stream based on the brightness information of the current image frame in the first video stream and the brightness information of the current image frame in the second video stream comprises:

obtaining the brightness-fused image by fusing pixels of the current image frame in the first video stream with respective pixels of the current image frame in the second video stream based on the brightness information of the current image frame in the first video stream and the brightness information of the current image frame in the second video stream.

28. The control method for a fluorescence endoscopy system according to claim 20, wherein obtaining the scene-fused image by fusing the current image frame in the first video stream with the brightness-fused image based on the chroma information of the current image frame in the first video stream comprises:

assigning chroma information of pixels of the current image frame in the first video stream to respective pixels of the brightness-fused image as chroma values thereof.

29. The control method for a fluorescence endoscopy system according to claim 20, further comprising, in case of the first video stream being output in a RAW or RGB format, before the current image frame in the first video stream is fused with the current image frame in the second video stream,

converting the current image frame in the first video stream into a YUV or $YC_bC_r$ space.

30. The control method for a fluorescence endoscopy system according to claim 27, wherein fusing the pixels of the current image frame in the first video stream with the respective pixels of the current image frame in the second video stream based on the brightness information of the current image frame in the first video stream and the brightness information of the current image frame in the second video stream comprises:

deriving weights for brightness values of the pixels of the current image frame in the first video stream and weights for brightness values of the respective pixels of the current image frame in the second video stream from the brightness values of the pixels of the current image frame in the first video stream and the brightness values of the respective pixels of the current image frame in the second video stream, respectively, according to a predefined normal weight distribution as a function of brightness; and obtaining brightness value of pixels of the brightness-fused image by weighting the brightness values of the pixels of the current image frame in the first video stream and the brightness values of the respective pixels of the current image frame in the second video stream with the derived weights for the brightness values of the pixels of the current image frame in the first video stream and weights for the brightness values of the respective pixels of the current image frame in the second video stream, respectively.

31. The control method for a fluorescence endoscopy system according to claim 20, wherein the endoscope is a three-dimensional endoscope,

wherein capturing the visible-light scene images and near-infrared scene images of the tissue of interest and outputting the visible-light scene images in a form of the first video stream and the near-infrared scene images in a form of the second video stream comprises:
capturing first visible-light scene images, second visible-light scene images, first near-infrared scene images and second near-infrared scene images of the tissue of interest and outputting respectively in a form of a first visible-light video stream, a second visible-light video stream, a first near-infrared video stream and a second near-infrared video stream;
wherein determining whether the current brightness value of the first video stream lies within the predefined first target brightness range comprises:
determining whether a current brightness value of the first visible-light video stream and/or a current brightness value of the second visible-light video stream lie(s) within the predefined first target brightness range;
wherein determining whether the current brightness value of the second video stream lies within the predefined second target brightness range comprises:
determining whether a current brightness value of the first near-infrared video stream and/or a current brightness value of the second near-infrared video stream lie(s) within the predefined second target brightness range;
wherein obtaining the brightness-fused image by fusing the current image frame in the first video stream with the current image frame in the second video stream based on the brightness information of the current image frame in the first video stream and the brightness information of the current image frame in the second video stream comprises:
obtaining a first brightness-fused image by fusing a current image frame in the first visible-light video stream with a current image frame in the first near-infrared video stream based on brightness information of the current image frame in the first visible-light video stream and brightness information of the current image frame in the first near-infrared video stream and obtaining a second brightness-fused image by fusing a current image frame in the second visible-light video stream with a current image frame in the second near-infrared video stream based on brightness information of the current image frame in the second visible-light video stream and brightness information of the current image frame in the second visible-light video stream and brightness information of the current image frame in the second near-infrared video stream; and

wherein obtaining the scene-fused image by fusing the current image frame in the first video stream with the brightness-fused image based on the chroma information of the current image frame in the first video stream comprises:

obtaining a first scene-fused image by fusing the current image frame in the first visible-light video stream with the first brightness-fused image based on chroma information of the current image frame in the first visible-light video stream and obtaining a second scene-fused image by fusing the current image frame in the second visible-light video stream with the second brightness-fused image based on chroma information of the current image frame in the second visible-light video stream.

32. A storage medium, storing thereon a computer program, which, when executed by a processor, implements the control method of any one of claims 20 to 31.

Fig. 1

Scene Fusion Module
500

Central
Controller 800

Endoscope
Control
Module
400

First Control
Unit 410

Second Control
Unit 420

Endoscope
Drive
Module 300

First Drive
Unit 310

Second Drive
Unit 320

Illumin
ation
Module
200

Illumi
nation
Contro
ller
220

Mode Switching
Unit 221

Power Control
Unit 222

Light Source Unit
210

Fig. 2

Fig. 3

Fig. 4

Fig. 5

Fig. 6

EP 4 248 835 A1

Weight

Fig. 7

Fig. 8

IN SECOND MODE, PROVIDING VISIBLE LIGHT AND NEAR-INFRARED LIGHT FOR ILLUMINATING TISSUE OF INTEREST — S1

CAPTURING VISIBLE-LIGHT SCENE IMAGES AND NEAR-INFRARED SCENE IMAGES OF TISSUE OF INTEREST AND OUTPUT THEM IN FORM OF FIRST VIDEO STREAM AND SECOND VIDEO STREAM, RESPECTIVELY — S2

WHETHER CURRENT BRIGHTNESS VALUE OF 1ST VIDEO STREAM LIES WITHIN PREDEFINED FIRST TARGET BRIGHTNESS RANGE AND/OR CURRENT BRIGHTNESS VALUE OF 2ND VIDEO STREAM LIES IS WITHIN PREDEFINED 2ND TARGET BRIGHTNESS RANGE — S3

YES

OBTAINING BRIGHTNESS-FUSED IMAGE BY FUSING CURRENT IMAGE FRAME IN FIRST VIDEO STREAM WITH CURRENT IMAGE FRAME IN SECOND VIDEO STREAM BASED ON BRIGHTNESS INFORMATION OF CURRENT IMAGE FRAME IN FIRST VIDEO STREAM AND BRIGHTNESS INFORMATION OF CURRENT IMAGE FRAME IN SECOND VIDEO STREAM — S4

OBTAINING SCENE-FUSED IMAGE BY FUSING CURRENT IMAGE FRAME IN FIRST VIDEO STREAM WITH BRIGHTNESS-FUSED IMAGE BASED ON CHROMA INFORMATION OF CURRENT IMAGE FRAME IN FIRST VIDEO STREAM — S5

Fig. 9

## INTERNATIONAL SEARCH REPORT

| | International application No. |
|---|---|
| | **PCT/CN2021/131950** |

**A. CLASSIFICATION OF SUBJECT MATTER**

A61B 1/06(2006.01)i

According to International Patent Classification (IPC) or to both national classification and IPC

**B. FIELDS SEARCHED**

Minimum documentation searched (classification system followed by classification symbols)

A61B 1

Documentation searched other than minimum documentation to the extent that such documents are included in the fields searched

Electronic data base consulted during the international search (name of data base and, where practicable, search terms used)

CNTXT; ENTXTC; ENTXT; VEN; CNKI; CNABS: 荧光, 内窥镜, 照明, 融合, 图像, 视频, 可见光, 近红外, 红外光, 场景, 曝光时间, 增益, 亮度, 色度, 传感器; endoscope, visible light, fluorescence, illumination, video stream, image, NIR, near infrared, exposure, time, gain, scene, chroma, sensor, fusion

**C. DOCUMENTS CONSIDERED TO BE RELEVANT**

| Category* | Citation of document, with indication, where appropriate, of the relevant passages | Relevant to claim No. |
|---|---|---|
| Y | CN 109618099 A (SHENZHEN INFINOVA LIMITED) 12 April 2019 (2019-04-12) description paragraphs 82-199 | 1-32 |
| Y | CN 103300812 A (INSTITUTE OF AUTOMATION, CHINESE ACADEMY OF SCIENCES) 18 September 2013 (2013-09-18) description paragraphs 24-47 | 1-32 |
| Y | CN 111818707 A (ZHEJIANG HEALNOC TECHNOLOGY CO., LTD.) 23 October 2020 (2020-10-23) description, paragraphs 47-98 | 1-32 |
| Y | CN 110811498 A (CHANGCHUN INSTITUTE OF OPTICS, FINE MECHANICS AND PHYSICS, CHINESE ACADEMY OF SCIENCES) 21 February 2020 (2020-02-21) description, paragraphs 35-42 | 1-32 |
| Y | CN 110893095 A (SHANGHAI YISI MEDICAL IMAGING EQUIPMENT CO., LTD.) 20 March 2020 (2020-03-20) description, paragraphs 51-71 | 1-32 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 January 2022** | **08 February 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/131950** |

### C.    DOCUMENTS CONSIDERED TO BE RELEVANT

| Category* | Citation of document, with indication, where appropriate, of the relevant passages | Relevant to claim No. |
| --- | --- | --- |
| Y | CN 109924938 A (SUZHOU BRAIN SPACE INFORMATION RESEARCH INSTITUTE, HUAZHONG UNIVERSITY OF SCIENCE AND TECHNOLOGY) 25 June 2019 (2019-06-25)<br>    description, paragraphs 33-53 | 1-32 |
| Y | CN 111803013 A (SHENZHEN BOSSUN HEALTH TECHNOLOGY CO., LTD.) 23 October 2020 (2020-10-23)<br>    description paragraphs 42-133 | 1-32 |
| A | CN 111948798 A (MICROPORT (SHANGHAI) MEDICAL ROBOT CO., LTD.) 17 November 2020 (2020-11-17)<br>    entire document | 1-32 |
| A | US 2012268573 A1 (SCHOENBORN KARL-HEINZ GUENTER et al.) 25 October 2012 (2012-10-25)<br>    entire document | 1-32 |
| A | KR 20180006668 A (EULJI UNIV INDUSTRY ACADEMY COOPERATION FOUNDATION) 19 January 2018 (2018-01-19)<br>    entire document | 1-32 |
| A | WO 2019191497 A1 (BLAZE BIOSCIENCE INC.) 03 October 2019 (2019-10-03)<br>    entire document | 1-32 |
| A | WO 2020198315 A1 (UNIV EAST CAROLINA) 01 October 2020 (2020-10-01)<br>    entire document | 1-32 |

Form PCT/ISA/210 (second sheet) (January 2015)

INTERNATIONAL SEARCH REPORT
Information on patent family members

International application No.

**PCT/CN2021/131950**

| Patent document cited in search report | | | Publication date (day/month/year) | Patent family member(s) | | | Publication date (day/month/year) |
|---|---|---|---|---|---|---|---|
| CN | 109618099 | A | 12 April 2019 | CN | 109618099 | B | 08 January 2021 |
| CN | 103300812 | A | 18 September 2013 | None | | | |
| CN | 111818707 | A | 23 October 2020 | None | | | |
| CN | 110811498 | A | 21 February 2020 | None | | | |
| CN | 110893095 | A | 20 March 2020 | WO | 2020052623 | A1 | 19 March 2020 |
| CN | 109924938 | A | 25 June 2019 | CN | 210330538 | U | 17 April 2020 |
| CN | 111803013 | A | 23 October 2020 | CN | 212326346 | U | 12 January 2021 |
| CN | 111948798 | A | 17 November 2020 | None | | | |
| US | 2012268573 | A1 | 25 October 2012 | WO | 2010142672 | A1 | 16 December 2010 |
| | | | | EP | 2440119 | A1 | 18 April 2012 |
| | | | | EP | 2440119 | B1 | 13 May 2015 |
| | | | | DE | 102009024943 | A1 | 16 December 2010 |
| | | | | EP | 2932892 | A1 | 21 October 2015 |
| | | | | US | 9433350 | B2 | 06 September 2016 |
| | | | | ES | 2544804 | T3 | 04 September 2015 |
| KR | 20180006668 | A | 19 January 2018 | KR | 101852181 | B1 | 25 April 2018 |
| WO | 2019191497 | A1 | 03 October 2019 | CN | 111970953 | A | 20 November 2020 |
| | | | | JP | 2021519446 | A | 10 August 2021 |
| | | | | KR | 20200138732 | A | 10 December 2020 |
| | | | | IL | 277530 | D0 | 30 November 2020 |
| | | | | TW | 201944955 | A | 01 December 2019 |
| | | | | AU | 2019243317 | A1 | 15 October 2020 |
| | | | | EP | 3773137 | A1 | 17 February 2021 |
| | | | | US | 2021015350 | A1 | 21 January 2021 |
| | | | | CA | 3093545 | A1 | 03 October 2019 |
| WO | 2020198315 | A1 | 01 October 2020 | CA | 3134066 | A1 | 01 October 2020 |
| | | | | US | 2020305721 | A1 | 01 October 2020 |

Form PCT/ISA/210 (patent family annex) (January 2015)